(19) Europäisches Patentamt
European Patent Office
Office européen des brevets

(11) **EP 3 677 235 A1**

(12) **EUROPEAN PATENT APPLICATION**
published in accordance with Art. 153(4) EPC

(43) Date of publication:
08.07.2020 Bulletin 2020/28

(51) Int Cl.:
*A61F 13/534* (2006.01)   *D04H 1/4382* (2012.01)
*D04H 1/46* (2012.01)

(21) Application number: 18882479.1

(22) Date of filing: 21.11.2018

(86) International application number:
PCT/JP2018/042938

(87) International publication number:
WO 2019/107238 (06.06.2019 Gazette 2019/23)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.11.2017 JP 2017228423
02.11.2018 JP 2018207605

(71) Applicant: Kao Corporation
Chuo-Ku
Tokyo 103-8210 (JP)

(72) Inventors:
• ITOI, Namie
Haga-gun
Tochigi 321-3497 (JP)
• YUYAMA, Aki
Haga-gun
Tochigi 321-3497 (JP)
• KANEKO, Masaya
Haga-gun
Tochigi 321-3497 (JP)

(74) Representative: Vossius & Partner
Patentanwälte Rechtsanwälte mbB
Siebertstrasse 3
81675 München (DE)

(54) **ABSORBENT BODY AND ABSORBENT ARTICLE**

(57) An absorbent member (4) of the present invention includes fiber clusters (11) containing synthetic fibers (11F) and includes water absorbing fibers (12F), and a plurality of the fiber clusters (11) are interlaced with each other or with the water absorbing fibers (12F). The content mass ratio of the fiber clusters (11) to the water absorbing fibers (12F) (fiber cluster ratio) is smaller in the side of a skin-facing surface (the side of a topsheet (2)) than in the side of a non-skin-facing surface (the side of a backsheet (3)) of the absorbent member (4). The fiber cluster (11) includes a main portion (110) formed by two opposite base surfaces (111) and a body surface (112) intersecting the base surfaces (111).

Fig. 2

**Description**

Technical Field

The present invention relates to an absorbent member that is used to be in direct or indirect contact with the skin and is suitable as the absorbent member for absorbent articles.

Background Art

An absorbent article such as disposable diapers and sanitary napkins typically includes a topsheet to be placed relatively close to the skin of a wearer, a backsheet to be placed relatively distant from the skin of a wearer, and an absorbent member between the sheets. The absorbent member typically includes water absorbing fibers such as wood pulp as a main component and further includes water absorbing polymer particles in many cases. The absorbent member used in absorbent articles has major problems of improving various properties including flexibility (cushioning properties), compression recovery properties, and shape retention properties.

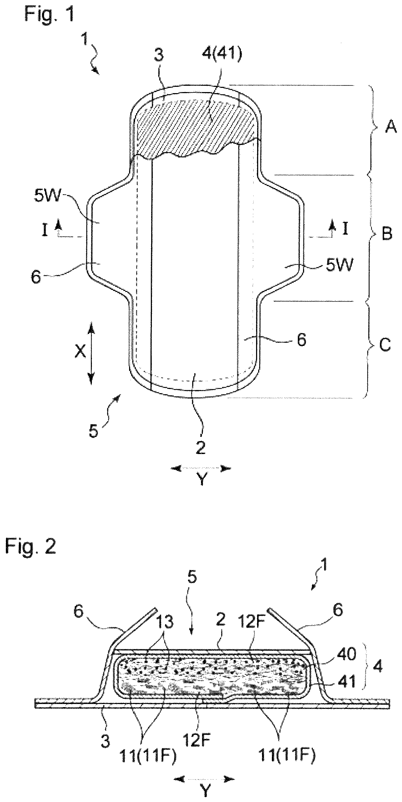

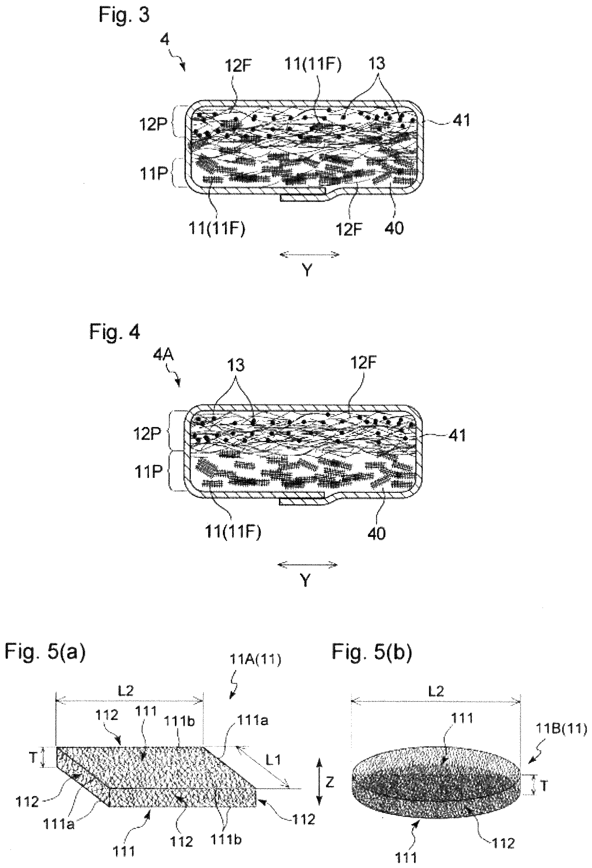

As a technique to improve absorbent members, for example, Patent Literature 1 discloses an absorbent member that includes nonwoven fabric pieces including heat-fusible fibers and having a three-dimensional structure that has been formed by bonding the fibers together and includes water absorbing fibers. The nonwoven fabric pieces having a three-dimensional structure are produced by cutting a nonwoven fabric into small pieces by using a cutting means such as a cutter mill system, thus have such an indefinite shape as shown in Fig. 1 and Fig. 3 in the literature due to the production method, and have substantially no portion considered to be flat. Patent Literature 1 discloses thermally fused nonwoven fabric pieces as a preferred embodiment of the absorbent member according to the literature. In the absorbent member according to Patent Literature 1, nonwoven fabric pieces have a three-dimensional structure, thus voids are formed in the absorbent member, and the recovery after absorption of water is improved. As a result, the water absorbability is considered to be improved.

Patent Literature 2 discloses a microweb including relatively dense microfiber nuclei and fibers or fiber bundles extending outward from the nuclei, and further discloses that a nonwoven web including a mixture of the microweb and wood pulp or water absorbing polymer particles is usable as the absorbent member for absorbent articles. This microweb is produced by divellicating or tearing a material sheet such as nonwoven fabric, thus has an indefinite shape as with the nonwoven fabric pieces according to Patent Literature 1, and has substantially no portion considered to be flat.

When an absorbent member containing a water absorbing polymer and interposed between a topsheet and a backsheet absorbs liquid to expand, such a sealed water absorbing polymer is prevent from swelling. In order to overcome the disadvantage, Patent Literature 3 discloses that a cushion layer having high compression/compression recovery properties and liquid permeability is interposed between the topsheet and the absorbent member, and further discloses that the cushion layer includes an aggregate of small pieces of nonwoven fabric. The literature also discloses that the cushion layer has a thickness of 10 to 40 mm. Patent Literature 3, however, does not specifically disclose the shape and the like of the nonwoven fabric small pieces used in the cushion layer.

Citation List

Patent Literatures

Patent Literature 1: US 2010/0174259 A
Patent Literature 2: US 4813948 B
Patent Literature 3: JP 2003-52750 A

Summary of Invention

The present invention relates to an absorbent member used to be in direct or indirect contact with the skin and includes a skin-facing surface to be placed relatively close to the skin of a user using at the time of use and a non-skin-facing surface to be placed relatively distant from the skin of the user. The absorbent member of the present invention includes fiber clusters containing synthetic fibers and includes water absorbing fibers, and a plurality of the fiber clusters are interlaced with each other or with the water absorbing fibers. The content mass ratio of the fiber clusters to the water absorbing fibers is smaller in the skin-facing surface side than in the non-skin-facing surface side.

The present invention is also an absorbent article including the absorbent member of the present invention.

Brief Description of Drawings

[Fig. 1] Fig. 1 is a partially broken plan view schematically showing a skin-facing surface (topsheet) side of an exemplary sanitary napkin as an embodiment of the absorbent article of the present invention.

[Fig. 2] Fig. 2 is a schematic cross-sectional view showing a cross section taken along line I-I in Fig. 1.

[Fig. 3] Fig. 3 is an enlarged cross-sectional view of only the absorbent member shown in Fig. 2.

[Fig. 4] Fig. 4 is a cross-sectional view of another embodiment of the absorbent member of the present invention corresponding to Fig. 3.

[Fig. 5] Fig. 5(a) and Fig. 5(b) are schematic perspective views of a main portion in a fiber cluster of the present invention.

[Fig. 6] Fig. 6 is an illustrative view of a production method of the fiber clusters of the present invention.

[Fig. 7] Fig. 7(a) is an electron micrograph (observation magnification: $25\times$) of an example of the fiber cluster of the present invention; and Fig. 7(b) is a view schematically showing the fiber cluster in the electron micrograph as a fiber cluster included in the absorbent member shown in Fig. 2.

[Fig. 8] Fig. 8 is an illustrative view of the measurement method of a surface diffusion area.

Description of Embodiments

The absorbent members according to Patent Literatures 1 and 2 include synthetic fiber aggregates, which have indefinite shapes as described above and have various shapes and sizes. Hence, when such aggregates are mixed with wood pulp or the like, a uniform mixture is difficult to produce, and intended effects may not be achieved. In addition, the synthetic fiber aggregates disclosed in the literatures are produced by cutting a nonwoven fabric mainly containing synthetic fibers into small pieces or by divellicating or tearing such a nonwoven fabric and thus is supposed to have a randomly roughened surface. In an absorbent member containing many synthetic fiber aggregates having such an entirely roughened surface, a plurality of synthetic fiber aggregates are entangled with each other over the entire face by a comparatively strong bonding force, and consequently the degree of freedom of the movement of synthetic fiber aggregates is markedly restricted. On this account, the absorbent member is unlikely to have clearance through which body fluid passes and has poor liquid absorbability. When all the synthetic fiber aggregates included in an absorbent member are fusion bonded to each other as in a preferred embodiment of the absorbent member according to Patent Literature 1, the movement of the fiber aggregates is restricted, thus the absorbent member has higher hardness as a whole, and the liquid absorbability may further deteriorate.

When including such synthetic fiber aggregates as described in Patent Literatures 1 and 2, the absorbent member has a larger distance among constituent fibers than that of a common absorbent member without such fiber aggregates, and thus the drawability of liquid on the absorbent member surface into the absorbent member (liquid drawability) may deteriorate. When such a cushion layer including synthetic fiber aggregates and having a considerable thickness as described in Patent Literature 3 is placed on an absorbent member, the above-mentioned liquid absorbability further deteriorates unfortunately.

The present invention is therefore intended to provide an absorbent member that has high cushioning properties, is unlikely to twist, has excellent liquid drawability, and can improve wearing comfort when included in an absorbent article and to provide an absorbent article including the absorbent member.

An absorbent member of the present invention and an absorbent article including the absorbent member of the present invention will now be described on the basis of preferred embodiments thereof with reference to drawings. Fig. 1 and Fig. 2 show a sanitary napkin 1 as an embodiment of the absorbent article of the present invention. The napkin 1 includes an absorbent member 4 that is to absorb and retain a body fluid, a liquid permeable topsheet 2 that is placed on a skin-facing surface of the absorbent member 4 and is to come into contact with the skin of a wearer, and a sparingly liquid permeable backsheet 3 that is placed on a non-skin-facing surface of the absorbent member 4. As shown in Fig. 1, the napkin 1 has a longitudinal direction X coincident with the front-to-rear direction of a wearer and extending from the abdominal side of a wearer via the crotch to the dorsal side and a lateral direction Y orthogonal thereto, and is divided in the longitudinal direction X into three regions, a longitudinal center region B including an excretory-facing region (excretion point) to face the excretory part such as the pudendum of a wearer, a front region A located closer to the abdominal side (front side) of a wearer than the excretory-facing region, and a rear region C located closer to the dorsal side (rear side) of a wearer than the excretory-facing region.

In the present description, a "skin-facing surface" is one face of an absorbent article or a constituent member thereof (for example, an absorbent member 4) and faces the skin of a wearer at the time of wearing of the absorbent article or is the face relatively close to the skin of a wearer, and a "non-skin-facing surface" is the other face of an absorbent article or a constituent member thereof and is the face opposite to the skin of a wearer at the time of wearing of the absorbent article or is the face relatively distant from the skin of a wearer. In the present description, "at the time of wearing" means a condition in which a typical, appropriate wearing position or a normal wearing position of the absorbent article is maintained.

As shown in Fig. 1, the napkin 1 includes an absorbent main body 5 oblong in the longitudinal direction X and a pair of wings 5W, 5W extending outward in the lateral direction Y from the corresponding sides along the longitudinal direction X of the longitudinal center region B in the absorbent main body 5. The absorbent main body 5 is a main part of the napkin 1, includes the topsheet 2, the backsheet 3, and the absorbent member 4, and is divided in the longitudinal direction X into three regions, the front region A, the longitudinal center region B, and the rear region C.

When an absorbent article has wings as in the napkin 1, the longitudinal center region in the absorbent article of the present invention means a region having wings in the longitudinal direction (length direction, X-direction in the drawing) of the absorbent article. In the napkin 1 as an example, the longitudinal center region is a region between the base of one wing 5W along the longitudinal direction X and the base of the other wing 5W along the longitudinal direction X. In an absorbent article without wings, the longitudinal center region means a middle region when the absorbent article is trisected in the longitudinal direction.

In the napkin 1, the absorbent member 4 includes a liquid-absorbing absorbent core 40 and a liquid permeable core-wrap sheet 41 wrapping the outer surface of the absorbent core 40. As with the absorbent main body 5, the absorbent core 40 has an oblong shape in the longitudinal direction X in such a planar view as shown in Fig. 1, the length direction of the absorbent core 40 is coincident with the longitudinal direction X of the napkin 1, and the width direction of the absorbent core 40 is coincident with the lateral direction Y of the napkin 1. The absorbent core 40 and the core-wrap sheet 41 may be joined with an adhesive such as a hot melt adhesive.

As described above, when the absorbent member 4 as an embodiment of the absorbent member of the present invention is included in such an absorbent article as the napkin 1, the absorbent member is used to be in indirect contact with the skin of a human or to be indirect contact with the skin through a member such as the backsheet 3. The absorbent member has a skin-facing surface (a face facing the topsheet 2) to be placed relatively close to the skin of a user (wearer wearing the napkin 1) at the time of use and a non-skin-facing surface (a face facing the backsheet 3) to be placed relatively distant from the skin of the user. The absorbent member also has a longitudinal direction X coincident with the front-to-rear direction of a wearer wearing the napkin 1 and a lateral direction Y orthogonal thereto and is divided in the longitudinal direction X into three regions, a front region A, a longitudinal center region B, and a rear region C. The absorbent member 4 may be used to be in indirect contact with the skin as described above or may be used to be in direct contact with the skin through no member such as a sheet.

In the napkin 1, the core-wrap sheet 41 is a single continuous sheet having a width that is twice or more and three times or less the dimension of the absorbent core 40 in the lateral direction Y. As shown in Fig. 2, the core-wrap sheet overlies the whole region of the skin-facing surface of the absorbent core 40 and extends from the respective side edges along the longitudinal direction X of the absorbent core 40 outward in the lateral direction Y, and the extension portions are folded under the absorbent core 40 and overlie the whole region of the non-skin-facing surface of the absorbent core 40. In the present invention, the core-wrap sheet is not limited to such a single sheet. For example, a core-wrap sheet may include two sheets, a single skin-facing core-wrap sheet overlying the skin-facing surface of the absorbent core 40 and a single non-skin-facing core-wrap sheet that is separated from the skin-facing core-wrap sheet and overlies the non-skin-facing surface of the absorbent core 40.

As shown in Fig. 2, the topsheet 2 overlies the whole region of the skin-facing surface of the absorbent member 4. The backsheet 3 overlies the whole region of the non-skin-facing surface of the absorbent member 4 and extends from the respective side edges along the longitudinal direction X of the absorbent member 4 outward in the lateral direction Y to form side flaps together with the side sheets 6 described later. In the napkin 1, the side flap includes a member extending from the absorbent member 4 outward in the lateral direction Y. The backsheet 3 and each side sheet 6 are joined together in an extension portion from the corresponding side edge along the longitudinal direction X of the absorbent member 4, by a known joining means such as an adhesive, heat sealing, and ultrasonic sealing. Each of the topsheet 2 and the backsheet 3 may be joined to the absorbent member 4 with an adhesive. As the topsheet 2 or the backsheet 3, various materials conventionally used in absorbent articles such as sanitary napkins can be used without any limitation. For example, as the topsheet 2, a single-layer or multilayer nonwoven fabric, a porous film, or the like can be used. As the backsheet 3, a moisture permeable resin film or the like can be used.

As shown in Fig. 1, the side flaps greatly protrude outward in the lateral direction Y in the longitudinal center region B, and accordingly, a pair of extending wings 5W, 5W are provided on both lateral sides along the longitudinal direction X of the absorbent main body 5. In such a planar view as shown in Fig. 1, each wing 5W has a substantially trapezoidal shape, and the lower base (the base longer than the upper base) is located on the corresponding side of the absorbent main body 5. The non-skin-facing surface thereof has a wing adhesion portion (not shown in the drawings) for fixing the wing 5W to clothes such as shorts. The wings 5W are used to be folded onto the non-skin-facing surface (the outer surface) of the crotch portion of clothes such as shorts. The wing adhesion portions are covered with release sheets (not shown in the drawings) made from a film, a nonwoven fabric, paper, or the like, before use. On both sides along the longitudinal direction X of the skin-facing surface of the absorbent main body 5 or the skin-facing surface of the topsheet 2, a pair of side sheets 6, 6 are provided over substantially the entire length in the longitudinal direction X of the absorbent main body 5 so as to overlap with both lateral sides along the longitudinal direction X of the absorbent member 4 in a planar view. The pair of side sheets 6, 6 are joined to other members including the topsheet 2 by a known joining means such as an adhesive, on joining lines (not shown in the drawings) each extending in the longitudinal direction X.

A main characteristic part of the napkin 1 is, for example, the absorbent member 4, specifically, the absorbent core 40 as the main constituent of the absorbent member 4. The absorbent core 40 is characterized by including fiber clusters 11 containing fibers (synthetic fibers) 11F in addition to water absorbing fibers 12F as shown in Fig. 2. In the absorbent core 40, the plurality of water absorbing fibers 12F are not accumulated but are each independently included, and each fiber can be separately extracted from the absorbent core 40, whereas each fiber cluster 11 is an integrated fiber aggregate in which a plurality of fibers 11F are intentionally accumulated into a cluster and can be included in the absorbent core 40 while the shape as the fiber aggregate is constantly maintained. The fiber clusters 11 mainly contribute to improvements in flexibility, cushioning properties, compression recovery properties, shape retention properties, and the like of the absorbent core 40. The water absorbing fibers 12F mainly contribute to improvements in liquid absorbability, shape retention properties, and the like of the absorbent core 40. The absorbent core 40 is considered to be substantially the absorbent member 4 itself, and the absorbent core 40 will be described as the absorbent member 4 in the following description unless otherwise specified. In other words, the present invention encompasses an absorbent member including no core-wrap sheet but including only an absorbent core, and in such a case, the absorbent member means the absorbent core.

In the present description, a "fiber cluster" is a fiber aggregate in which a plurality of fibers aggregate into one body. Examples of the shape of the fiber cluster include a sheet piece prepared by dividing a synthetic fiber sheet having a certain size. In particular, the fiber cluster is preferably a nonwoven fabric piece prepared by cutting a nonwoven fabric selected as the synthetic fiber sheet into a predetermined size and shape.

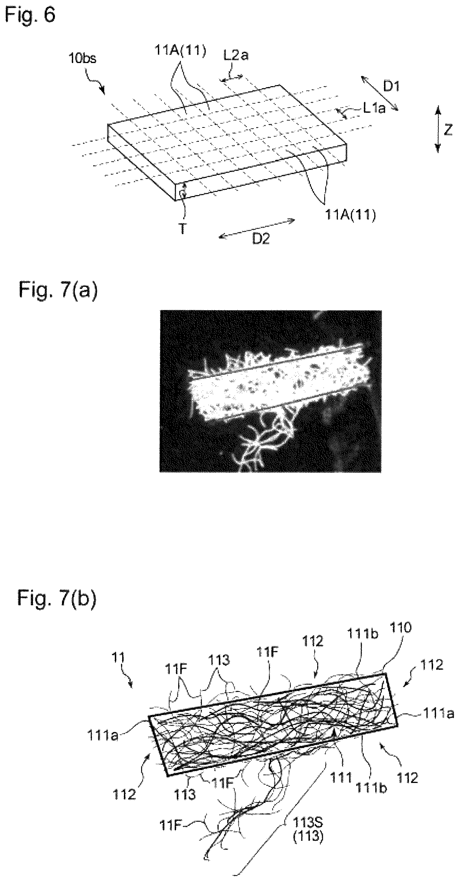

As described above, a sheet piece-like fiber cluster as a preferred embodiment of the fiber cluster of the present invention is not produced by accumulating a plurality of fibers into the sheet piece but is produced by cutting a fiber sheet (preferably a nonwoven fabric) having a larger size than that of the sheet piece (see Fig. 6). The plurality of fiber clusters included in the absorbent member of the present invention are a plurality of sheet piece-like fiber clusters having a more definite shape than those produced by a conventional art as in Patent Literatures 1 and 2.

In the absorbent core 40, the plurality of fiber clusters 11 are interlaced with each other or with the water absorbing fibers 12F. In the absorbent core 40 of the present embodiment, a plurality of fiber clusters 11 are bonded by entanglement of constituent fibers (fibers 11F, 12F) in the absorbent core 40 to form a single fiber cluster continuous body. Together with the interlacement of a plurality of fiber clusters 11, the fiber clusters 11 may be interlaced with the water absorbing fibers 12F and be bonded together. Typically, a plurality of water absorbing fibers 12F are also interlaced with each other. At least some of the plurality of fiber clusters 11 included in the absorbent core 40 are interlaced with other fiber clusters 11 or the water absorbing fibers 12F. In the absorbent core 40, all the plurality of fiber clusters 11 included therein may be interlaced with each other to form a single fiber cluster continuous body in some cases, and a plurality of fiber cluster continuous bodies may be mixed in a nonbonding state in some cases.

The fiber clusters 11 have excellent flexibility or the like. The absorbent core 40 of the present invention includes such fiber clusters 11, and the fiber clusters 11 are bonded to each other or to the water absorbing fibers 12F by interlacement. Hence, the absorbent core 40 has more excellent responsivity to an external force and has excellent flexibility, cushioning properties, and compression recovery properties. For example, when included in an absorbent article, the absorbent core 40 of the present invention flexibly deforms in response to external forces (for example, a body pressure of a wearer wearing the absorbent article) applied in various directions and allows the absorbent article to come into close contact with the body of a wearer with satisfactory fitting properties. Such an excellent deformation-recovery properties of the absorbent core 40 can be expressed not only when the absorbent core 40 is compressed but also when the absorbent core is distorted. In other words, the absorbent core 40 included in the napkin 1 is placed while interposed between the thighs of a wearer wearing the napkin 1, and thus the absorbent member 4 may be distorted around a virtual rotation axis extending in the longitudinal direction X by movement of the thighs when the wearer walks. Even in such a case, the absorbent core 40, which has high deformation-recovery properties, easily deforms/recovers with respect to such an external force as to promote the distortion from the thighs, thus is unlikely to twist, and can impart, to the napkin 1, high fitting properties to the body of a wearer.

In the absorbent core 40, the fiber clusters 11 are interlaced with each other or with the water absorbing fibers 12F. In the description, "interlacement" of fiber clusters 11 and the like includes the following configuration A.

Configuration A: Fiber clusters 11 and the like are bonded without fusion but with entanglement of constituent fibers 11F of the fiber clusters 11.

Configuration B: When an absorbent core 40 is in a natural state (without any external force), fiber clusters 11 and the like are not bonded, but when an external force is applied to the absorbent core 40, fiber clusters 11 and the like can be bonded by entanglement of constituent fibers 11F. In the configuration, "an external force being applied to an absorbent core 40" is that, for example, a deformation force is applied to an absorbent core 40 when an absorbent article including the absorbent core 40 is worn.

As described above, in the absorbent core 40, fiber clusters 11 are bonded to other fiber clusters 11 or to water absorbing fibers 12F by fiber entanglement or "interlacement" as in the configuration A, and fiber clusters 11 can be bonded to other fiber clusters 11 or to water absorbing fibers 12F by interlacement as in the configuration B. Such bonding by the fiber interlacement is an important point to more effectively exert the above advantageous effects of the absorbent core 40. The absorbent core preferably has the "interlacement" in the configuration A from the viewpoint of shape retention properties.

In the absorbent core 40, all the bonding manner through fiber clusters 11 is not necessarily limited to "interlacement", and the absorbent core 40 may partly include additional bonding manners other than the interlacement, such as joining with an adhesive.

However, in the residual part in the absorbent core 40 except "fusion through fiber clusters 11" formed in the absorbent core 40 by integration with other members such as known leak-proof grooves of an absorbent article, or in other words, in the absorbent core 40 itself, bonding between fiber clusters 11 or bonding between fiber clusters 11 and water absorbing fibers 12F is preferably formed only by "fiber interlacement".

From the viewpoint of more certainly allowing the absorbent core 40 to exert the above advantageous effects, the total number of "fiber clusters 11 bonded by interlacement" in the configuration A and "fiber clusters 11 capable of being interlaced" in the configuration B is preferably 50% or more, more preferably 70% or more, and even more preferably 80% or more relative to the total number of the fiber clusters 11 in the absorbent core 40.

From the same viewpoint, the number of fiber clusters 11 with "interlacement" in the configuration A is preferably 70% or more, particularly 80% or more of the total number of the fiber clusters 11 bonded to other fiber clusters 11 or to water absorbing fibers 12F.

The napkin 1 is also characterized by the arrangement of fiber materials (fiber clusters 11, water absorbing fibers 12F) in the absorbent core 40, in addition to the fiber clusters 11 included. In other words, in the absorbent core 40, the content mass ratio of the fiber clusters 11 to the water absorbing fibers 12F (hereinafter also called "fiber cluster ratio") is smaller in the skin-facing surface side (in the side of the topsheet 2) than in the non-skin-facing surface side (in the side of the backsheet 3), as shown in Fig. 2 and Fig. 3. In other words, in the absorbent core 40, the water absorbing fibers 12F are localized in the skin-facing surface side, and the fiber clusters 11 (synthetic fibers 11F) are localized in the non-skin-facing surface side.

As described above, in the absorbent core 40, the skin-facing surface side of the absorbent core 40 as the side firstly receiving a body fluid excreted from a wearer wearing the napkin 1 is a portion having a lower fiber ratio (including a fiber cluster ratio of 0% by mass), that is, a "water absorbing fiber rich portion 12P" containing a larger amount of water absorbing fibers 12F than in other portions of the absorbent core 40 (see Fig. 3). Hence, the skin-facing surface of the absorbent core 40 has a higher liquid drawing force (capillary force), and the absorbent core 40 has satisfactory liquid absorbability. In contrast, the non-skin-facing surface side of the absorbent core 40 is a portion having a higher fiber cluster ratio (including a case containing no water absorbing fibers but containing only fiber clusters), that is, a "fiber cluster rich portion 11P" containing a larger amount of fiber clusters 11 than in other portions of the absorbent core 40 (see Fig. 3). Hence, the absorbent core 40 can sufficiently exert advantageous effects mainly by fiber clusters 11, such as improvement effects of cushioning properties and anti-twisting properties. In the fiber cluster rich portion 11P, fiber clusters 11 are preferably uniformly distributed at a high density in the whole portion 11P from the viewpoint of more certainly allowing the portion 11P to exert intended advantageous effects.

The fiber cluster ratio, that is, the content mass ratio of fiber clusters 11 to water absorbing fibers 12F is calculated in the following manner. In a portion to be measured in the thickness direction of an absorbent member 4 (absorbent core 40), or in the direction orthogonal to the skin-facing surface or the non-skin-facing surface of an absorbent member 4 (absorbent core 40) (for example, in the skin-facing surface side of an absorbent member 4), the contents (masses) of the fiber clusters 11 and the water absorbing fibers 12F contained in the portion to be measured are determined, then the resulting content of the fiber clusters 11 is divided by the content of the water absorbing fibers 12F, and the result in percentage is the fiber cluster ratio of the portion to be measured.

The fiber cluster ratio in the skin-facing surface side of the absorbent core 40 or in the water absorbing fiber rich portion 12P is preferably 140% by mass or less, more preferably 70% by mass or less, and even more preferably 30% by mass or less from the viewpoint of quick absorption of a body fluid into the absorbent core 40. The lower limit of the fiber cluster ratio in the water absorbing fiber rich portion 12P is preferably 10% by mass, more preferably 5% by mass, and even more preferably 0% by mass.

The fiber cluster ratio in the non-skin-facing surface side of the absorbent core 40 or in the fiber cluster rich portion 11P is preferably 70% by mass or more, more preferably 140% by mass or more, and even more preferably 170% by mass or more, provided that the fiber cluster ratio is larger than that in the skin-facing surface side. The upper limit of the fiber cluster ratio in the non-skin-facing surface side of the absorbent core 40 is not specifically limited, and the content of the water absorbing fibers 12F in the non-skin-facing surface side may be 0% by mass. In this case, the fiber cluster ratio is an infinite value because the denominator in the calculation formula is 0, but in the present description, the case may be expressed as "only fiber clusters" or as a fiber cluster occupancy of 100% by mass as described later in place of the fiber cluster ratio.

As described above, the napkin 1 is characterized by the absorbent core 40 in which the water absorbing fibers 12F are localized in the skin-facing surface side and the fiber clusters 11 (synthetic fibers 11F) are localized in the non-skin-facing surface side. As an index indicating such characteristic distribution of fiber clusters 11, the proportion of the mass of the fiber clusters 11 relative to the total mass of fiber clusters 11 and water absorbing fibers 12F (hereinafter also called "fiber cluster occupancy") can be used in place of the fiber cluster ratio.

The fiber cluster occupancy is calculated in the following manner. In a portion to be measured in the thickness direction of an absorbent member 4 (absorbent core 40) or in the direction orthogonal to the skin-facing surface or the non-skin-facing surface of an absorbent member 4 (absorbent core 40) (for example, in the skin-facing surface side of an absorbent member 4), the contents (masses) of the fiber clusters 11 and the water absorbing fibers 12F contained in the portion to be measured are determined, then the resulting mass of the fiber clusters 11 is divided by the total mass of the water absorbing fibers 12F and the fiber clusters 11, and the result in percentage is the fiber cluster occupancy of the portion to be measured.

From the viewpoint of more certainly exerting the above advantageous effects, the fiber cluster occupancy in each portion of the absorbent core 40 is preferably set as follows, provided that the occupancy is smaller in the non-skin-facing surface side than in the skin-facing surface side.

The fiber cluster occupancy in the skin-facing surface side of the absorbent core 40 or in the water absorbing fiber rich portion 12P is preferably 60% by mass or less, more preferably 30% by mass or less, and even more preferably 20% by mass or less. The lower limit of the fiber cluster occupancy in the water absorbing fiber rich portion 12P is preferably 10% by mass, more preferably 5% by mass, and even more preferably 0% by mass.

The fiber cluster occupancy in the non-skin-facing surface side of the absorbent core 40 or in the fiber cluster rich portion 11P is preferably 50% by mass or more, more preferably 60% by mass or more, and even more preferably 70% by mass or more and is preferably 100% by mass or less, more preferably 95% by mass or less, and even more preferably 85% by mass or less.

The difference in fiber cluster occupancy between the fiber cluster rich portion 11P and the water absorbing fiber rich portion 12P, where the latter is subtracted from the former, is preferably 15% by mass or more, more preferably 50% by mass or more, and even more preferably 80% by mass or more.

As for the range of "fiber cluster occupancy" in a fiber cluster rich portion 11P or a water absorbing fiber rich portion 12P and the range of "fiber cluster ratio" in a fiber cluster rich portion 11P or a water absorbing fiber rich portion 12P of an absorbent core 40 having a unity as a whole, the absorbent core 40 is bisected in the thickness direction, and a portion on the skin-facing surface is regarded as the fiber cluster rich portion 11P, whereas a portion on the non-skin-facing surface is regarded as the water absorbing fiber rich portion 12P.

In the description, "an absorbent core having a unity as a whole" means that the absorbent core is integrally formed and excludes the structure in which a plurality of separately formed absorbent core layers are stacked. In the latter structure, one absorbent core layer can be separated from the other absorbent core layer, and the layers are not an inseparable unity and are not integrated. Specific examples of the absorbent core having a unity as a whole include an absorbent core in which all the forming materials (fiber clusters 11, water absorbing fibers 12F) of an absorbent core 40 are fiber stacked at once. Each absorbent core 40 in the absorbent member 4 shown in Fig. 3 and the absorbent member 4A shown in Fig. 4 described later includes two portions having different fiber cluster occupancies or fiber cluster ratios (water absorbing fiber rich portion 12P, fiber cluster rich portion IIP), but constituent fibers 12F, 11F are interlaced with each other on the interface between the two portions 12P, 11P, and thus the absorbent core has a unity as a whole. In contrast, in an absorbent core 40 in a modified embodiment of the absorbent member 4A described later, constituent fibers 12F, 11F are not substantially interlaced with each other on the interface between two portions 12P, 11P as separated layers, and thus the absorbent core has no unity as a whole.

In the present invention, the water absorbing fiber rich portion 12P preferably contains only water absorbing fibers 12F, but when a water absorbing fiber rich portion 12P also contains fiber clusters 11, such an absorbent core 40 can generally improve the effect derived from the fiber clusters 11 and thus is preferred. In particular, when a water absorbing fiber rich portion 12P contains fiber clusters 11 and water absorbing fibers 12F as a mixture, and the fiber clusters 11 are interlaced or can be interlaced with the water absorbing fibers 12F, the water absorbing fibers 12F in the water absorbing fiber rich portion 12P firstly absorbs a body fluid such as menstrual blood, and subsequently, the body fluid is supplied to space in the fiber cluster rich portion 11P. Hence, such a structure is preferred. In addition, in the fiber cluster rich portion 11P, the fiber clusters 11 are preferably interlaced with water absorbing fibers 12F because such a structure is likely to exert the above effects.

In the absorbent core 40, the fiber cluster occupancy or the fiber cluster ratio gradually increases from the skin-facing surface (topsheet 2) toward the non-skin-facing surface (backsheet 3) as shown in Fig. 3. In other words, in the absorbent core 40, the number of water absorbing fibers 12F gradually decreases from the skin-facing surface toward the non-skin-facing surface. In short, in the thickness direction of the absorbent core 40, on and near the skin-facing surface of the absorbent core 40, fiber clusters 11 are absent or present at the minimum fiber cluster occupancy or the minimum fiber cluster ratio in the absorbent core 40, whereas on or near the non-skin-facing surface of the absorbent core 40, fiber clusters 11 are present at the maximum fiber cluster occupancy or the maximum fiber cluster ratio in the absorbent core 40.

The arrangement of the fiber materials (fiber clusters 11, water absorbing fibers 12F) in the absorbent core 40 is not limited to such an arrangement as shown in Fig. 3, that is, the arrangement in which the fiber cluster occupancy or the fiber cluster ratio gradually changes from one face in the thickness direction of the absorbent core 40 toward the other face, and can be appropriately modified without departing from the technical spirit of the present invention. Fig. 4 shows an absorbent member 4A as another embodiment of the absorbent member of the present invention corresponding to Fig. 3. The absorbent member 4A will be described mainly about components different from the above absorbent member 4, and a similar component is indicated by the same reference number and is not described. To components not specifically described, the description for the absorbent member 4 (absorbent core 40) will be appropriately applied.

The absorbent member 4A shown in Fig. 4 is divided in the thickness direction into two layered portions (water absorbing fiber rich portion 12P, fiber cluster rich portion 11P) having different fiber cluster occupancies or fiber cluster ratios. In the two portions, or in each of the water absorbing fiber rich portion 12P as the skin-facing surface side and the fiber cluster rich portion 11P as the non-skin-facing surface side, the fiber cluster occupancy or the fiber cluster ratio is not necessarily constant in the thickness direction of the absorbent member 4A. For each of the portions 11P, 12P, the difference in fiber cluster occupancy between a skin-facing surface side portion and a non-skin-facing surface side portion bisected in the thickness direction is preferably 30% by mass or less and more preferably 10% by mass or less. In the absorbent member 4A, the fiber cluster occupancy or the fiber cluster ratio preferably greatly changes on the interface between the water absorbing fiber rich portion 12P and the fiber cluster rich portion 11P. In the embodiment shown in Fig. 4, the interface between the portions is the center of the absorbent member 4A in the thickness direction, that is, the absorbent member 4A is bisected in the thickness direction into the water absorbing fiber rich portion 12P and the fiber cluster rich portion 11P, but the position of the interface can be appropriately set, and the portions can have different thicknesses.

When an absorbent core 40 is divided as layers into a water absorbing fiber rich portion 12P and a fiber cluster rich portion 11P as in the absorbent member 4A shown in Fig. 4, the water absorbing fiber rich portion 12P is preferably from the skin-facing surface of the absorbent core 40 inward in the thickness direction of the absorbent core 40 to 20 to 80% of the thickness of the absorbent core 40, more preferably to 30 to 70% of the thickness. The fiber cluster rich portion 11P is preferably from the non-skin-facing surface of the absorbent core 40 inward in the thickness direction of the absorbent core 40 to 20 to 80% of the thickness of the absorbent core 40, more preferably to 30 to 70% of the thickness.

When an absorbent core 40 is divided as layers into a water absorbing fiber rich portion 12P and a fiber cluster rich portion 11P as in the absorbent member 4A shown in Fig. 4, each thickness of the water absorbing fiber rich portion 12P and the fiber cluster rich portion 11P is preferably 0.5 mm or more and more preferably 1 mm or more and is preferably 10 mm or less, more preferably 5 mm or less, and even more preferably 4 mm or less.

The thickness of each portion of the absorbent core 40 is determined in the following manner. The total thickness of an absorbent core 40 (absorbent member 4), the thickness of a napkin 1, and the like can also be determined in accordance with the following method.

<Measurement method of thickness>

An absorbent core (absorbent member) is allowed to stand on a horizontal place so as not to have wrinkles or creases, and from the absorbent core, a portion to be measured (for example, the skin-facing surface side or the non-skin-facing surface side of the absorbent core) is cut out as a measurement sample. The thickness of the measurement sample is determined under a load of 5 cN/cm$^2$. Specifically, the thickness is determined, for example, by using a thickness gauge, PEACOCK DIAL UPRIGHT GAUGES R5-C (manufactured by OZAKI MFG. CO. LTD.). In the measurement, between the tip of the thickness gauge and the measurement sample, a round or square plate in a planar view (an acrylic plate having a thickness of about 5 mm) adjusted to give a load of 5 cN/cm$^2$ is placed, and the thickness is measured. The thickness was measured at 10 points, and the average is calculated to give a thickness.

In the absorbent member 4A, the water absorbing fiber rich portion 12P is bonded to the fiber cluster rich portion 11P on the interface therebetween by interlacement of constituent fibers (the constituent fibers 11F of the fiber clusters 11, the water absorbing fibers 12F). In other words, on the interface between the portions, the water absorbing fibers 12F in the water absorbing fiber rich portion 12P as the skin-facing surface side are interlaced with the fiber clusters 11 (fibers 11F) in the fiber cluster rich portion 11P as the non-skin-facing surface side, accordingly the portions are relatively loosely bonded to each other, and thus the absorbent member 4A (absorbent core 40) has a unity as a whole.

Modified embodiments of the absorbent member 4A shown in Fig. 4 include an absorbent member in which a water absorbing fiber rich portion 12P as the skin-facing surface side is not bonded to a fiber cluster rich portion 11P as the non-skin-facing surface side, and the absorbent member is also encompassed by the present invention. To produce the modified embodiment of the absorbent member 4A, first, a layer corresponding to the water absorbing fiber rich portion 12P and a layer corresponding to the fiber cluster rich portion 11P are separately produced, and the layers are stacked to give the absorbent member. In the modified embodiment of the absorbent member 4A produced by the above method, constituent fibers 11F and 12F in the portions are hardly interlaced with each other, thus the absorbent core 40 has no unity as a whole, and the water absorbing fiber rich portion 12P can be easily separated from the fiber cluster rich portion 11P.

Such an absorbent member 4 as shown in Fig. 3, in which the fiber cluster occupancy or the fiber cluster ratio gradually changes from one face in the thickness direction of the absorbent member toward the other face, specifically has the following advantages: the ratio of water absorbing fibers and fiber clusters gradually changes in the thickness direction of the absorbent member, thus the interlacement through the fiber clusters is likely to be maintained in the thickness direction even when an external force is applied to the absorbent member, and the cushioning properties of the absorbent member is likely to be satisfactory maintained at the time of use. Such an absorbent member 4A as shown in Fig. 4, which includes, in the thickness direction, two portions having different fiber cluster occupancies or fiber cluster ratios, specifically has the following advantages: the skin-facing surface side and the non-skin-facing surface side of the absorbent member are easily designed to have independent functions.

The absorbent member 4 or 4A (absorbent core 40) can be produced by using a known fiber stacking apparatus with a rotating drum by a common method. The specific arrangement of fiber materials (fiber clusters 11, water absorbing fibers 12F) as shown in Fig. 3 or Fig. 4 can be achieved, for example, by appropriately setting the stacking order of fiber clusters 11 and water absorbing fibers 12F on the rotating drum in the production method using the fiber stacking apparatus.

The absorbent core 40 may include additional components other than the fiber clusters 11 and the water absorbing fibers 12F, and examples of the additional component include a water absorbing polymer. Reference number 13 in the drawings is a water absorbing polymer. As the water absorbing polymer, a particulate polymer as shown in the figures is typically used, and a fibrous polymer may be used. When used, the particulate water absorbing polymer may have any shape of a spherical shape, a cluster shape, an ellipsoid shape, and an indefinite shape. The water absorbing polymer preferably has an average particle size of 10 $\mu$m or more and more preferably 100 $\mu$m or more and preferably 1,000 $\mu$m or less and more preferably 800 $\mu$m or less. As the water absorbing polymer, typically, a polymer or a copolymer of acrylic acid or an alkali metal acrylate can be used. Examples thereof include polyacrylic acid, salts thereof, polymethacrylic acid, and salts thereof.

In the absorbent core 40, the water absorbing polymer 13 is contained in the skin-facing surface side of the absorbent core 40 and accordingly contained in the water absorbing fiber rich portion 12P, as shown in Fig. 3. As described above, the skin-facing surface side of the absorbent core 40, which is specifically intended to have excellent liquid drawability, contains the water absorbing polymer 13 having excellent water absorbability, and thus the liquid drawability on the skin-facing surface of the absorbent core 40 can be further improved in combination with the water absorbing fiber rich portion 12P as the skin-facing surface side, that is, in cooperation with a relatively low fiber cluster occupancy or fiber cluster ratio.

In the absorbent core 40, the water absorbing polymer 13 may be contained in a portion other than the skin-facing surface side (water absorbing fiber rich portion 12P) of the absorbent core 40 or in the fiber cluster rich portion 11P. Considering that the water absorbing polymer 13 is contained in the absorbent core 40 mainly for improvement in liquid drawability of the skin-facing surface side of the absorbent core 40, it may not be very significant that the non-skin-facing surface side (fiber cluster rich portion 11P) of the absorbent core 40 intentionally contains the water absorbing polymer 13. In addition, when the non-skin-facing surface side (fiber cluster rich portion 11P) of the absorbent core 40 intentionally contains the water absorbing polymer 13, the advantageous effects (such as improvement effects of cushioning properties and anti-twisting properties) by the fiber cluster rich portion 11P may not be satisfactory exerted. In consideration of the above, a portion other than the skin-facing surface side (water absorbing fiber rich portion 12P) in the absorbent core 40, specifically, for example, the fiber cluster rich portion 11P preferably has a lower content of the water absorbing polymer 13 than that of the skin-facing surface side, and the content may be 0.

In the absorbent core 40, the content of the water absorbing polymer 13 is preferably 5% by mass or more and more preferably 10% by mass or more and is preferably 60% by mass or less and more preferably 40% by mass or less relative to the total mass of a dried absorbent core 40.

In the absorbent core 40, the basis weight of the water absorbing polymer 13 is preferably 10 g/m$^2$ or more and more preferably 30 g/m$^2$ or more and is preferably 100 g/m$^2$ or less and more preferably 70 g/m$^2$ or less.

A main feature of the absorbent core 40 is the outer shape of the fiber cluster 11. Fig. 5 shows two typical outer shapes of the fiber cluster 11. A fiber cluster 11A shown in Fig. 5(a) has a quadratic prism shape, more specifically, a rectangular parallelepiped shape, whereas a fiber cluster 11B shown in Fig. 5(b) has a disk shape. The fiber clusters 11A and 11B are common in having two opposite base surfaces 111 and body surfaces 112 connecting the two base surfaces 111. Each of the base surfaces 111 and the body surfaces 112 substantially has no unevenness at the level of unevenness evaluation on the surface of an article mainly containing this kind of fibers.

The fiber cluster 11A having a rectangular parallelepiped shape in Fig. 5(a) have six flat surfaces. Of the six surfaces, two opposite surfaces having the maximum area are the base surfaces 111, and the remaining four surfaces are the body surfaces 112. The base surfaces 111 intersect with, more specifically, are orthogonal to the body surfaces 112.

The disk-shaped fiber cluster 11B in Fig. 5(b) has two opposite flat surfaces having a round shape in a planar view and a curved circumferential surface connecting the flat surfaces. The two flat surfaces are the base surfaces 111, and the circumferential surface is the body surface 112.

The fiber clusters 11A and 11B are common in that the body surfaces 112 have a quadrangular shape in a planar view, more specifically, a rectangular shape.

The plurality of fiber clusters 11 contained in the absorbent core 40 differ from the nonwoven fabric piece or the microweb according to Patent Literatures 1 and 2 as an indefinite fiber aggregate in that each fiber cluster is a "definite fiber aggregate" having two opposite base surfaces 111 and body surfaces 112 connecting the base surfaces 111 as the fiber clusters 11A and 11B shown in Fig. 5. In other words, when looking through a fiber cluster 11 arbitrarily selected from among the fiber clusters 11 contained in the absorbent core 40 (for example, observed under an electron microscope), the perspective shape of the fiber cluster 11 varies with observation angles, and a single fiber cluster 11 gives a large number of perspective shapes. Each of the plurality of fiber clusters 11 in the absorbent core 40 has, as one of the large number of perspective shapes, a particular perspective shape having two opposite base surfaces 111 and body surfaces 112 connecting the base surfaces 111. The plurality of nonwoven fabric pieces or the microweb contained in the absorbent member according to Patent Literatures 1 and 2 does not substantially has such a "surface" as the base surface 111 or the body surface 112, that is, a spreading portion, has various outer shapes, and has no "definite shape".

As described above, when a plurality of fiber clusters 11 contained in the absorbent core 40 are "definite fiber aggregates" formed by base surfaces 111 and body surfaces 112, the uniform dispersibility of the fiber clusters 11 in the absorbent core 40 is improved as compared with such indefinite fiber aggregates as described in Patent Literatures 1 and 2. Hence, the effects (improvement effects of the flexibility, the cushioning properties, the compression recovery properties, and the like of the absorbent member), which should be exerted when such fiber aggregates as fiber clusters 11 are contained in the absorbent core 40, are stably achieved. In particular, such a fiber cluster 11 having a rectangular parallelepiped shape as shown in Fig. 5(a), which has, as the outer surfaces, six surfaces including two base surfaces 111 and four body surfaces 112, can come into contact with other fiber clusters 11 or water absorbing fibers 12F more frequently than such a disk-shaped fiber cluster 11 having three outer surfaces as shown in Fig. 5(b). Accordingly, the interlacing property is improved, and the shape retention properties or the like can be improved.

In the fiber cluster 11, the total area of two base surfaces 111 is preferably larger than the total area of body surfaces 112. In other words, in the fiber cluster 11A having a rectangular parallelepiped shape in Fig. 5(a), the sum of the areas of two base surfaces 111 is larger than the sum of the areas of four body surfaces 112, and in the disk-shaped fiber cluster 11B in Fig. 5(b), the sum of the areas of two base surfaces 111 is larger than the area of the body surface 112 forming the circumferential surface of the disk-shaped fiber cluster 11B. In each of the fiber clusters 11A and 11B, the base surface 111 has the largest area of the plurality of surfaces of the fiber cluster 11A or 11B.

Such a fiber cluster 11 as the "definite fiber aggregate" defined by two base surfaces 111 and body surfaces 112 intersecting with the base surfaces 111 can be achieved by a different production method from the conventional techniques. A preferred production method of the fiber cluster 11 is, as shown in Fig. 6, by cutting a material fiber sheet lObs (a sheet having the same composition as the fiber cluster 11 but having a larger size than the fiber cluster 11) as the material by using a cutting means such as a cutter into a definite shape. A plurality of fiber clusters 11 produced have a more definite shape and size than those produced by the conventional techniques as described in Patent Literatures 1 and 2. Fig. 6 is an illustrative view of the production method of the fiber clusters 11A having a rectangular parallelepiped shape in Fig. 5(a), and the dotted lines in Fig. 6 indicate cutting lines. The absorbent core 40 includes a plurality of fiber clusters 11 having a uniform shape and size and produced by cutting a fiber sheet into a definite shape as described above. The material fiber sheet lObs is preferably a nonwoven fabric as described above.

The fiber cluster 11A having a rectangular parallelepiped shape in Fig. 5(a) is produced by cutting a material fiber sheet lObs in a first direction D1 and a second direction D2 intersecting (more specifically orthogonal to) the first direction D1 into predetermined lengths as shown in Fig. 6. Each of the directions D1 and D2 is a certain direction on the surface direction of the sheet lObs, and the sheet lObs is cut along the thickness direction Z orthogonal to the surface direction. On a plurality of fiber clusters 11A having a rectangular parallelepiped shape prepared by cutting the material fiber sheet lObs into what is called dice in this manner, typically, a cut surface, that is, a surface in contact with a cutting means such as a cutter when the sheet lObs is cut is the body surface 112, and a non-cut surface, that is, a surface not in contact with the cutting means is the base surface 111. The base surface 111 is the top or back surface (a surface orthogonal to the thickness direction Z) of the sheet lObs and has the largest area of a plurality of surfaces of the fiber cluster 11A as described above.

The above description about the fiber cluster 11A can be basically applied to the disk-shaped fiber cluster 11B in Fig. 5(b). The substantial difference from the fiber cluster 11A is only the cutting pattern of a material fiber sheet lObs, and to cut a sheet lObs into a definite shape to prepare fiber clusters 11B, the sheet lObs can be cut into a round shape corresponding to the shape of the fiber cluster 11B in a planar view.

The outer shape of the fiber cluster 11 is not limited to those shown in Fig. 5, and each of the base surface 111 and the body surface 112 may be a flat surface without curve as the surfaces 111 and 112 in Fig. 5(a) or may be a curved surface as the body surface 112 (the circumferential surface of the disk-shaped fiber cluster 11B) in Fig. 5(b). The base surface 111 and the body surface 112 may have the same shape and the same size.

As described above, two types of surfaces (the base surface 111 and the body surface 112) of the fiber cluster 11 (11A or 11B) can be classified into a cut surface (body surface 112) formed by cutting a material fiber sheet lObs by a cutting means such as a cutter when fiber clusters 11 are prepared and a non-cut surface (base surface 111) that is the original surface of the sheet lObs and is not in contact with the cutting means. Due to the difference between the cut surface and the non-cut surface, the body surface 112 as the cut surface characteristically has a larger number of fiber ends in a unit area than that of the base surface 111 as the non-cut surface. In the description, the "fiber end" means an end in the length direction of a constituent fiber 11F of the fiber cluster 11. Although the base surface 111 as the non-cut surface typically includes fiber ends, the body surface 112, which is a cut surface formed by cutting the material fiber sheet lObs, includes, on the whole body surface 112, a large number of fiber ends as cut ends of constituent fibers 11F formed by cutting and thus has a larger number of fiber ends in a unit area than that of the base surface 111.

The fiber ends on each surface (the base surface 111 and body surface 112) of the fiber cluster 11 have advantages when the fiber cluster 11 forms interlacement with other fiber clusters 11 or water absorbing fibers 12F contained in an absorbent core 40. Typically, a larger number of fiber ends in a unit area improves interlacing property and thus can improve various characteristics such as shape retention properties of an absorbent core 40. As described above, the surfaces of a fiber cluster 11 have different numbers of fiber ends in a unit area, and the number of fiber ends in a unit area satisfies the relation "body surface 112 > base surface 111". Hence, the interlacing property through a fiber cluster 11 with other fibers (other fiber clusters 11 and water absorbing fibers 12F) varies with surfaces of the fiber cluster 11, and the body surface 112 has higher interlacing property than that of the base surface 111. In other words, the bonding by interlacement with other fibers through the body surface 112 is stronger than that through the base surface 111, and a single fiber cluster 11 has a difference in bonding force between the base surface 111 and the body surface 112. Typically, fibers bonded with a stronger bonding force have a lower degree of freedom of the movement, and a resulting absorbent core 40 has higher strength (shape retention properties) as a whole but is likely to have lower softness.

As described above, each of a plurality of fiber clusters 11 contained in an absorbent core 40 is interlaced with other peripheral fibers (other fiber clusters 11 and water absorbing fibers 12F) with two kinds of bonding forces, and accordingly, the absorbent core 40 has appropriate softness and strength (shape retention properties) together. In addition, when an absorbent core 40 having such excellent characteristics is used as the absorbent member in an absorbent article in a usual manner, the absorbent article can provide wearing comfort to a wearer thereof and can effectively prevent such a disadvantage that the absorbent core 40 is broken by external forces including a body pressure of a wearer at the time of wearing.

In particular, on the fiber cluster 11 (11A or 11B) shown in Fig. 5, the two base surfaces 111 have a larger total area than the total area of the body surfaces 112 as described above. This means that the base surfaces 111 having a relatively small number of fiber ends in a unit area and accordingly having a relatively small interlacing property with other fibers have a larger total area than that of the body surfaces 112 having characteristics opposite thereto. The fiber cluster 11 (11A or 11B) shown in Fig. 5 is therefore likely to be prevented from being interlaced with other peripheral fibers (other fiber clusters 11 and water absorbing fibers 12F) as compared with fiber clusters uniformly having fiber ends on the whole surface. Even if interlaced with other peripheral fibers, the fiber cluster is likely to be interlaced with a comparatively small bonding force, thus is unlikely to form a large aggregate, and can impart excellent flexibility to an absorbent core 40.

In contrast, the nonwoven fabric piece or the microweb according to Patent Literatures 1 and 2, which is produced, for example, by cutting a material fiber sheet with a cutter such as a mill cutter into an indefinite shape as described above, is not a definite sheet piece-like fiber cluster having such "surfaces" as the base surface 111 and the body surface 112. Moreover, in the production, an external force of the cutting treatment is applied to the whole fiber cluster, thus fiber ends of constituent fibers are randomly formed on the whole fiber cluster, and the above-descried advantageous effects by the fiber ends are unlikely to be sufficiently achieved.

From the viewpoint of more reliably achieve the above advantageous effects by the fiber ends, the ratio of the number of fiber ends in a unit area on the base surface 111 (non-cut surface), $N_1$, to the number of fiber ends in a unit area on the body surface 112 (cut surface), $N_2$, $N_1/N_2$, is preferably 0 or more and more preferably 0.05 or more and is preferably 0.90 or less and more preferably 0.60 or less, provided that $N_1 < N_2$. More specifically, $N_1/N_2$ is preferably 0

or more and 0.90 or less and more preferably 0.05 or more and 0.60 or less.

The number of fiber ends in a unit area on the base surface 111, $N_1$, is preferably 0 fiber end portions/mm$^2$ or more and more preferably 3 fiber end portions /mm$^2$ or more and is preferably 8 fiber end portions /mm$^2$ or less and more preferably 6 fiber end portions /mm$^2$ or less.

The number of fiber ends in a unit area on the body surface 112, $N_2$, is preferably 5 fiber end portions /mm$^2$ or more and more preferably 8 fiber end portions /mm$^2$ or more and is preferably 50 fiber end portions /mm$^2$ or less and more preferably 40 fiber end portions /mm$^2$ or less.

The number of fiber ends in a unit area on the base surface 111 or the body surface 112 is determined by the following method.

<Measurement method of the number of fiber ends in a unit area on each surface of a fiber cluster>

A member (fiber cluster) containing fibers to be measured (measurement piece) is attached to a sample table with a double-sided adhesive paper tape (NICETACK NW-15 manufactured by Nichiban Co., Ltd.). Next, the measurement piece is coated with platinum. For the coating, an ion sputtering apparatus, E-1030 (trade name) manufactured by Hitachinaka Seiki Co., Ltd is used, and the sputtering time is set at 120 seconds. Cut surfaces (a base surface and a body surface) of the measurement piece are observed under an electron microscope, JCM-6000 manufactured by JEOL at a magnification of 100×. In an observation image at a magnification of 100×, a rectangular region having a length of 1.2 mm and a width of 0.6 mm is set at any position on a surface to be measured (a base surface or a body surface), and the observation angle and the like are adjusted so that the area of the rectangular region is 90% or more of the area of the observation image. The number of fiber ends in the rectangular region is then counted. When a fiber cluster measurement surface is smaller than 1.2 mm × 0.6 mm in an observation image at a magnification of 100×, and the proportion of the area of the rectangular region in the total area of the observation image is less than 90%, the observation magnification is set at more than 100×, and the number of fiber ends in the rectangular region on the surface to be measured is counted in a similar manner. In the description, "fiber ends" of which the number is to be counted are ends in the length direction of constituent fibers of a fiber cluster, and even when portions other than the ends in the length direction of the constituent fibers (intermediate portions in the length direction) extend from a surface to be measured, the intermediate portions in the length direction are excluded from the counting. The number of fiber ends in a unit area on a fiber cluster measurement surface (a base surface or a body surface) is then calculated in accordance with the following formula. For each of 10 fiber clusters, the number of fiber ends in a unit area on each of the base surface and the body surface is counted in accordance with the above procedure, and the average value of the plurality of counted values is defined as the number of fiber ends in a unit area on the surface to be measured.

$$\text{The number of fiber ends in a unit area on a fiber cluster measurement surface (a base surface or a body surface) (number/mm}^2) = \text{the number of fiber ends contained in a rectangular region (}1.2 \times 0.6 \text{ mm)/the area of a rectangular region (}0.72 \text{ mm}^2)$$

When the base surface 111 of a fiber cluster 11 has a rectangular shape in a planar view as the fiber cluster 11A shown in Fig. 5(a), the short side 111a of the rectangular shape is preferably equal to or shorter than the thickness of the absorbent core 40 containing the fiber cluster 11 (11A) from the viewpoint of improvement in uniform dispersibility of fiber clusters 11 in an absorbent core 40. The ratio of the length of the short side 111a to the thickness of the absorbent core 40, the former/the latter, is preferably 0.03 or more and more preferably 0.08 or more and is preferably 1 or less and more preferably 0.5 or less.

The thickness of the absorbent core 40 is preferably 1 mm or more and more preferably 2 mm or more and is preferably 15 mm or less, more preferably 10 mm or less, and even more preferably 6 mm or less. The thickness of an absorbent core 40 is determined by the method described above.

The dimensions and the like of each portion of the fiber cluster 11 (11A or 11B) are preferably set to the following values. The dimensions of each portion of the fiber cluster 11 can be determined on the basis of an electron micrograph or the like at the time of the specifying operation of the outer shape of the fiber cluster 11 described later.

When the base surface 111 has a rectangular shape in a planar view as shown in Fig. 5(a), the length L1 of the short side 111a is preferably 0.3 mm or more and more preferably 0.5 mm or more and is preferably 10 mm or less and more preferably 6 mm or less. The length L2 of the long side 111b of a base surface 111 having a rectangular shape in a planar view is preferably 0.3 mm or more and more preferably 2 mm or more and is preferably 30 mm or less and more preferably 15 mm or less.

When the base surface 111 has the largest area of the plurality of surfaces of the fiber cluster 11 as shown in Fig. 5, the length L2 of the long side 111b is equal to the maximum dimension of the fiber cluster 11, and the maximum dimension is equal to the diameter of the base surface 111 having a round shape in a planar view of the disk-shaped fiber cluster 11B.

The ratio of the length L1 of the short side 111a to the length L2 of the long side 111b, L1/L2, is preferably 0.003 or more and more preferably 0.025 or more and is preferably 0.5 or less.

The thickness T of the fiber cluster 11, that is, the length T between two opposite base surfaces 111 is preferably 0.1 mm or more and more preferably 0.3 mm or more and is preferably 10 mm or less and more preferably 6 mm or less.

The absorbent core 40 is preferably dynamically isotropic in order to help the fiber clusters 11 to exert advantageous effects on each surface of the absorbent core 40. To achieve the effects, the fiber clusters 11 are preferably uniformly distributed at a high density in the whole absorbent core 40. From such a viewpoint, in any 10-mm square unit region of the absorbent core 40 in projection views in two directions orthogonal to each other, a plurality of fiber clusters 11 preferably overlap with each other. In the description, "projection views in two directions orthogonal to each other" typically include a projection view of an absorbent core (absorbent member) in the thickness direction (i.e. when an absorbent core is observed from the skin-facing surface or non-skin-facing surface thereof) and a projection view in a direction orthogonal to the thickness direction (i.e. when an absorbent core is observed from the side face).

Fig. 7(a) is an electron micrograph of an example of the fiber cluster of the present invention; and Fig. 7(b) is a view schematically showing the fiber cluster 11 in the electron micrograph. A plurality of fiber clusters 11 contained in an absorbent core 40 can include, as shown in Fig. 7, a fiber cluster that has a main portion 110 and an extending fiber portion 113 containing fibers 11F extending outward from the main portion 110 and having a lower fiber density (a smaller number of fibers in a unit area) than that of the main portion 110. The absorbent core 40 can include a fiber cluster 11 having no extending fiber portion 113, that is, a fiber cluster 11 having only a main portion 110. The extending fiber portion 113 is a kind of fiber ends on each surface (base surface 111 or body surface 112) of the fiber cluster 11 described above and is, of the fiber ends, fiber ends extending outward from surfaces of the fiber cluster 11.

The main portion 110 is formed by the above-described two opposite base surfaces 111 and the body surfaces 112 connecting the base surfaces 111. The main portion 110 mainly constitutes the fiber cluster 11 and defines the definite outer shape of the fiber cluster 11, and various properties of the fiber cluster 11, such as high flexibility, cushioning properties, and compression recovery properties, mainly arise from the main portion 110 basically. Meanwhile, the extending fiber portion 113 mainly contributes to an improvement in interlacing property between a plurality of fiber clusters 11 or between fiber clusters 11 and water absorbing fibers 12F contained in an absorbent core 40 to directly improve the shape retention properties of the absorbent core 40 and affects uniform dispersibility or the like of fiber clusters 11 in an absorbent core 40 to indirectly enhance advantageous effects by the main portion 110.

The main portion 110 has a higher fiber density or a larger number of fibers in a unit area than that of the extending fiber portion 113. Typically, the main portion 110 itself has a uniform fiber density. The proportion of the main portion 110 in the total mass of the fiber cluster 11 is typically at least 40% by mass or more, preferably 50% by mass or more, more preferably 60% by mass or more, and even more preferably 85% by mass or more. The main portion 110 and the extending fiber portion 113 can be distinguished by the following operation of specifying outer shapes.

The operation to specify the outer shape of the main portion 110 of a fiber cluster 11 contained in an absorbent core 40 can be performed by identifying the "boundary" between the main portion 110 and the other portions while the difference in fiber density of the absorbent core 40 (the difference in the number of fibers in a unit area) or the difference in type or fiber diameter of fibers are specifically observed. The main portion 110 has a higher fiber density than that of the peripheral extending fiber portions 113. In addition, synthetic fibers as the constituent fibers of the main portion 110 typically differ in quality and/or size from water absorbing fibers 12F (typically cellulose fibers). Hence, even when an absorbent core 40 contains a large number of fiber clusters 11 and water absorbing fibers 12F as a mixture, the boundary can be easily identified by specifically observing the above points. The boundary identified as above is the margin of a base surface 111 or a body surface 112, and the base surface 111 and the body surface 112 are specified by the boundary identification operation. Consequently, a main portion 110 can be specified. Such a boundary identification operation can be performed by observing an object (absorbent member 4) under an electron microscope, as necessary, at a plurality of observation angles. In particular, when the fiber cluster 11 contained in an absorbent core 40 is such a fiber cluster 11A or 11B shown in Fig. 5 that "the total area of two base surfaces 111 is larger than the total area of body surfaces 112", especially, when the base surface 111 has the largest area of the fiber cluster 11, the base surface 111 having a large area can be relatively easily specified, and thus the outer shape of the main portion 110 can be smoothly specified.

The extending fiber portion 113 includes constituent fibers 11F of the main portion 110, and the constituent fibers extend outward from at least one surface of the base surfaces 111 and the body surfaces 112 defining the outer surface of the main portion 110, as shown in Fig. 7. Fig. 7 is a surface view of a fiber cluster 11 viewed from the side of the base surface 111 (the surface having the largest area of the plurality of surfaces on the fiber cluster 11), and a large number of fibers 11F extend from a body surface 112 intersecting the base surface 111 to form an extending fiber portion 113.

The extending fiber portion 113 may have any shape. The extending fiber portion 113 may be composed of a single fiber 11F or may be composed of a plurality of fibers 11F as the extending fiber bundle 113S described later. The extending fiber portion 113 includes an end in the length direction of a fiber 11F extending from the main portion 110 and, in addition to such a fiber end, may include a portion other than the ends in the length direction of a fiber 11F (intermediate portion in the length direction). In other words, in a fiber cluster 11, the ends in the length direction of a constituent fiber 11F may be in the main portion 110, and the other portion or the intermediate portion in the length direction may extend (protrude) outward from the main portion 110 in a loop shape. In such a case, the extending fiber portion 113 includes the loop-like protrusion portion of a fiber 11F.

A main role of the extending fiber portion 113 is, as described above, to interlace a plurality of fiber clusters 11 contained in an absorbent core 40 with each other or to interlace fiber clusters 11 with water absorbing fibers 12F. Typically, when an extending fiber portion 113 has a larger extending length from the main portion 110, an extending fiber portion 113 has a larger thickness, or a single fiber cluster 11 has a larger number of extending fiber portions 113, substances interlaced through the extending fiber portion 113 are more strongly linked, and the interlacement is unlikely to be released. Hence, the intended effects of the present invention are exerted more stably.

In the case where the fiber cluster 11 is a fiber cluster obtained by cutting the raw material fiber sheet lObs into definite shapes as shown in Fig. 6, a relatively large number of extended fiber portions 113 are present in the body surface 112 formed by a cut surface of the fiber cluster 11, but in the base surface 111 that is a non-cut surface, there are no extended fiber portions 113, or if any, the number of extended fiber portions 113 is smaller than that in the body surface 112. The reason why extending fiber portions 113 are localized on body surfaces 112 as the cut surfaces is that many of the extending fiber portions 113 are "fuzz" generated by cutting a material fiber sheet. In other words, a body surface 112 formed by cutting a material fiber sheet lObs has been wholly rubbed by a cutting means such as a cutter at the time of the cutting, thus fuzz is likely to be formed from constituent fibers 11F of the sheet lObs, and the body surface is likely to have fuzz. Depending on types of the material fiber sheet, when cutting lines have small intervals or the cutting speed is set at low, extending fiber portions 113 are likely to be formed, and the length thereof can be controlled. In contrast, the base surfaces 111 as the non-cut surfaces are not rubbed by such a cutting means, and thus fuzz or extending fiber portions 113 are unlikely to be formed.

Intervals L1a (intervals in a first direction, see Fig. 6) and intervals L2a (intervals in a second direction, see Fig. 6) of the cutting lines at the time of cutting a material fiber sheet lObs are preferably 0.3 mm or more and more preferably 0.5 mm or more and are preferably 30 mm or less and more preferably 15 mm or less in order to help the formation of extending fiber portions 113 described above, and to provide fiber clusters 11 having such a necessary size as to express intended effects.

As shown in Fig. 7, the fiber cluster 11 has, as an extending fiber portion 113, an extending fiber bundle 113S extending outward from the main portion 110, more specifically, from the body surface 112, and containing a plurality of fibers 11F. At least one of the extending fiber portions 113 of the fiber cluster 11 can be the extending fiber bundle 113S. The extending fiber bundle 113S includes a plurality of gathered fibers 11F extending from the body surface 112 and is characterized by having a larger extending length from the body surface 112 than that of the extending fiber portion 113. The extending fiber bundle 113S can be on the base surface 111 but is typically on the body surface 112 as shown in Fig. 7, and the base surface 111 has no extending fiber bundle or a smaller number of extending fiber bundles than that on the body surface 112 even when extending fiber bundles are observed. The reason is the same as that why the extending fiber portions 113 are mainly on the body surface 112 as the cut surface is as described above.

When a fiber cluster 11 has such an extending fiber bundle 113S as to be called a long, thick, large-sized extending fiber portion 113, the interlacement between the fiber clusters 11 or between the fiber clusters 11 and water absorbing fibers 12F is further enhanced. As a result, the intended effects of the present invention due to the presence of the fiber clusters 11 are more stably exerted. The extending fiber bundle 113S is likely to be formed by cutting a material fiber sheet lObs in the above condition in which fuzz is likely to be formed (see Fig. 6).

The extending length of the extending fiber bundle 113S from the main portion 110, that is, the extending length from the body surface 112 (cut surface) is preferably 0.2 mm or more and more preferably 0.5 mm or more and is preferably 7 mm or less and more preferably 4 mm or less. The extending length of the extending fiber bundle 113S can be determined by the specifying operation of the outer shape of a fiber cluster 11 (boundary identification operation) described above. Specifically, for example, under a microscope manufactured by Keyence Corporation (at a magnification of 50×), a double-sided adhesive tape manufactured by 3M Company is attached onto the surface of a transparent acrylic sample table, and thereon, a fiber cluster 11 is placed and fixed. In accordance with the above outer shape specifying operation, the outer shape of the fiber cluster 11 is specified, then the extending length of a fiber 11F extending from the outer shape is measured, and the measured extending length is regarded as the extending length of an extending fiber bundle 113S.

In the extending fiber bundle 113S, a plurality of constituent fibers 11F thereof are preferably fusion bonded to each other. The fusion bond portion of the extending fiber bundle 113S typically has a larger dimension in a direction orthogonal to the length direction of the extending fiber bundle 113S (diameter when the fusion bond portion has a round cross section) than that of other portions (non-fusion bond portion) of the extending fiber bundle 113S. When the extending fiber bundle 113S has such a fusion bond portion as to be called a large diameter portion, the extending fiber bundle 113S itself has a higher strength, and this structure further enhances the interlacement between fiber clusters 11 or between fiber clusters 11 and water absorbing fibers 12F interlaced through the extending fiber bundle 113S. An extending fiber bundle 113S having a fusion bond portion has such advantages that the extending fiber bundle 113S itself has higher strength, shape retention properties, and the like not only when the extending fiber bundle 113S is in a dry state but also when the extending fiber bundle 113S absorbs water to be in a wet state. Due to such advantages, the advantageous effects by the presence of the fiber clusters 11 can be stably exerted not only when the absorbent core 40 is in a dry state but also when the absorbent core 40 absorbs body fluids excreted from a wearer, such as urine and menstrual blood, to be in a wet state. Such an extending fiber bundle 113S having the fusion bond portion can be produced, in the production process of fiber clusters 11 as shown in Fig. 6, that is, in the cutting process of a material fiber sheet IObs for fiber clusters 11, by using, as the material fiber sheet IObs, a "fiber sheet having fusion bond portions of constituent fibers".

The constituent fibers 11F of the fiber cluster 11 include synthetic fibers. The synthetic fibers used as the fibers 11F preferably have a lower water absorbability (poor water absorbability) than that of the water absorbing fibers 12F and are particularly preferably non-water absorbing synthetic fibers. The constituent fibers 11F of the fiber cluster 11 can include fiber components (for example, natural fibers) other than the synthetic fibers. When the constituent fibers 11F of the fiber cluster 11 include poorly hydrophilic fibers, preferably, non-water absorbing fibers, advantageous effects (improvement effects of shape retention properties, flexibility, cushioning properties, compression recovery properties, anti-twisting properties, and the like) due to the presence of the fiber cluster 11 are stably exerted not only when the absorbent core 40 is in a dry state but also when the absorbent core 40 absorbs water (body fluids such as urine and menstrual blood) to be in a wet state. In the fiber cluster 11, the content of the synthetic fibers as the constituent fibers 11F is preferably 90% by mass or more and more preferably 100% by mass relative to the total mass of the fiber cluster 11, that is, the fiber cluster 11 more preferably includes only synthetic fibers. In particular, when the synthetic fibers as the constituent fibers 11F are non-water absorbing fibers, the advantageous effects due to the presence of the fiber cluster 11 are even more stably exerted.

In the present description, the term "water absorbability" can be easily understood by a person skilled in the art in such a way that, for example, pulp has water absorbability. In a similar manner, thermoplastic fibers having poor water absorbability (especially non-water absorbability) can also be easily understood. Meanwhile, as for the degree of water absorbability of fibers, the relative difference in water absorbability of fibers can be compared by a moisture content determined by the method described later, and a more preferred range thereof can also be defined. Fibers having a higher moisture content have higher water absorbability. The water absorbing fibers preferably have a moisture content of 6% or more and more preferably 10% or more. Meanwhile, the synthetic fibers preferably have a moisture content of less than 6% and more preferably less than 4%. Fibers having a moisture content of less than 6% can be determined to be non-water absorbing fibers.

<Measurement method of moisture content>

The moisture content was determined in accordance with the moisture content test method in JIS P8203. In other words, a fiber sample was allowed to stand in a test room at a temperature of 40°C and a relative humidity of 80% RH for 24 hours, and then the weight W (g) of the fiber sample before the absolute dry treatment was determined in the room. The sample was then allowed to stand in an electric dryer (for example, manufactured by Isuzu Seisakusho, Co., Ltd.) at a temperature of 105 $\pm$ 2°C for 1 hour to perform absolute dry treatment of the fiber sample. After the absolute dry treatment, in a test room in a standard condition at a temperature of 20 $\pm$ 2°C and a relative temperature of 65 $\pm$ 2%, the fiber sample wrapped with Saran Wrap (registered trademark) manufactured by Asahi Kasei Corporation is placed in a glass desiccator (for example, manufactured by Tech-Jam) containing Si silica gel (for example, Toyotakako Co., Ltd.) and is allowed to stand until the temperature of the fiber sample reaches 20 $\pm$ 2°C. The constant weight W' (g) of the fiber sample is then determined, and the moisture content of the fiber sample is calculated in accordance with the following equation. Moisture content(%) = (W - W'/W') $\times$ 100

From the viewpoint of allowing the absorbent core 40 to similarly exert excellent effects on shape retention properties, flexibility, cushioning properties, compression recovery properties, anti-twisting properties, and the like in each of a dry condition and a wet condition, the fiber cluster 11 preferably has a three-dimensional structure in which a plurality of thermoplastic fibers are fusion bonded to each other.

To prepare such a fiber cluster 11 in which a plurality of fusion bond portions are three-dimensionally dispersed, the synthetic fibers used as the constituent fibers 11F of the fiber clusters 11 are preferably thermoplastic fibers. As described above, the extending fiber bundle 113S preferably has a fusion bond portion. When the constituent fibers 11F of the fiber clusters 11 are thermoplastic fibers, the extending fiber bundle 113S can have such a preferred structure.

In order to prepare the fiber cluster 11 in which a plurality of fusion bond portions are three-dimensionally dispersed, the material fiber sheet IObs (see Fig. 6) thereof can have a similar structure. Such a material fiber sheet lObs in which a plurality of fusion bond portions are three-dimensionally dispersed can be produced by subjecting a web or a nonwoven fabric mainly containing thermoplastic fibers to heat treatment such as hot air treatment, as described above.

Examples of the non-water absorbing synthetic resin (thermoplastic resin) preferred as the material of the constituent fibers 11F of the fiber cluster 11 include polyolefins such as polyethylene and polypropylene; polyesters such as polyethylene terephthalate; polyamides such as nylon 6 and nylon 66; and polyacrylic acid, polyalkyl methacrylate, polyvinyl chloride, and polyvinylidene chloride, and these resins can be used singly or in combination of two or more of them. The fiber 11F may be a monofilament of a single synthetic resin (thermoplastic resin) or a blend polymer of a mixture of two or more synthetic resins or may be a conjugated fiber. In the description, the conjugated fiber is a synthetic fiber (thermoplastic fiber) produced by conjugating two or more synthetic resins containing different components in a spinneret and simultaneously spinning the synthetic resins and has a structure in which a plurality of components continue in the length direction of the corresponding fibers and are bonded to each other in a single fiber. The structure of the conjugated fiber includes a core-sheath structure, a side-by-side structure, and the like and is not specifically limited.

The fiber cluster 11 preferably has a water contact angle of less than 90 degrees and particularly less than 70 degrees, from the viewpoint of further improving drawability of body fluid at the initial excretion. Such fibers can be produced by treatment of the above-described non-water absorbing synthetic fibers with a hydrophilizing agent in a common procedure. As the hydrophilizing agent, a common surfactant can be used.

<Measurement method of contact angle>

From a measurement object (an absorbent core), a fiber is sampled, and the contact angle of water to the fiber is measured. As the measurement device, an automatic contact angle meter, MCA-J, manufactured by Kyowa Interface Science Co., Ltd. is used. To measure the contact angle, deionized water is used. The liquid amount ejected from an inkjet type water droplet ejection unit (manufactured by Cluster Technology Co., Ltd., a pulse injector, CTC-25, having an ejection unit pore size of 25 μm) is set to 20 picoliters, and a water drop is dropped from directly above the fiber. The dropping state is recorded with a high-speed video recorder connected to a camera installed horizontally. The video recorder is preferably a personal computer integrated with a high-speed capturing apparatus from the viewpoint of subsequent image analysis. In the measurement, images are recorded every 17 msec. Of the recorded images, the first image in which a water drop reaches a fiber is subjected to image analysis using an accompanying software, FAMAS (ver. 2.6.2; analytical technique, droplet; analytical method, $\theta/2$; image processing algorithm, non-reflecting; image processing mode, frame; threshold level, 200; without curvature correction) to calculate the angle between the surface of the water drop in contact with air and the fiber, giving a contact angle. The fiber sampled from a measurement object is cut into a fiber length of 1 mm, and the cut fiber is placed on a sample table of a contact angle meter and is held horizontally. For a single fiber, contact angles are measured at different two sites. Contact angles of five fibers are measured to one decimal place, and the average of the measured values at 10 sites in total (rounded at a second decimal place) is defined as the water contact angle of the fibers. The measurement environment is a room temperature of 22 ± 2°C and a humidity of 65 ± 2% RH.

When an absorbent member (absorbent core) as the measurement object is used as a constituent member of another article such as an absorbent article and is fixed to another constituent member by means of an adhesive, fusing, or the like, and the absorbent member is taken out for evaluation, the adhesive strength of the fixing part is released by cold spraying or other methods in such a range as not to affect the contact angle of fibers, and then the absorbent article is taken out. The procedure is common in all the measurements in the present description.

As the water absorbing fibers 12F, water absorbing fibers conventionally used as the forming material of an absorbent member in this kind of absorbent article can be used. Examples of the water absorbing fibers include natural fibers such as wood pulps including softwood pulp and hardwood pulp and non-wood pulps including cotton pulp and hemp pulp; modified pulps such as cationized pulp and mercerized pulp; and regenerated fibers such as cupra and rayon, and these can be used singly or as a mixture of two or more of them. As described above, considering that the main role of the water absorbing fibers 12F is an improvement in liquid absorbability of an absorbent member 4, the water absorbing fibers 12F are preferably natural fibers or regenerated fibers (cellulose fibers).

In the absorbent member 4, the content mass ratio between the fiber clusters 11 and the water absorbing fibers 12F is not specifically limited and can be appropriately adjusted depending on types of the constituent fibers (synthetic fibers) 11F in the fiber clusters 11 and of the water absorbing fibers 12F, and the like. For example, when the constituent fibers 11F in the fiber clusters 11 are thermoplastic fibers (non-water absorbing synthetic fibers), and the water absorbing fibers 12F are water absorbing cellulose fibers, the content mass ratio between the fiber clusters 11 and the water absorbing fibers 12F, the former (fiber clusters 11)/the latter (water absorbing fibers 12F), is preferably 20/80 to 80/20 and more preferably 40/60 to 60/40, from the viewpoint of more certainly exerting intended effects of the present invention.

In the absorbent member 4, the content of the fiber clusters 11 is preferably 20% by mass or more and more preferably 40% by mass or more and is preferably 80% by mass or less and more preferably 60% by mass or less relative to the total mass of the dried absorbent member 4.

In the absorbent member 4, the content of the water absorbing fibers 12F is preferably 20% by mass or more and more preferably 40% by mass or more and is preferably 80% by mass or less and more preferably 60% by mass or less relative to the total mass of the dried absorbent member 4.

In the absorbent member 4, the basis weight of the fiber clusters 11 is preferably 32 $g/m^2$ or more and more preferably 80 $g/m^2$ or more and is preferably 640 $g/m^2$ or less and more preferably 480 $g/m^2$ or less.

In the absorbent member 4, the basis weight of the water absorbing fibers 12F is preferably 32 $g/m^2$ or more and more preferably 80 $g/m^2$ or more and is preferably 640 $g/m^2$ or less and more preferably 480 $g/m^2$ or less.

The absorbent member 4 can be produced in a similar manner to that of an absorbent member containing this kind of fiber material. The fiber cluster 11 can be produced as described above by cutting a material fiber sheet (a sheet having the same composition as the fiber cluster 11 and having a larger size than the fiber cluster 11) as the material by using a cutting means such as a cutter in two directions intersecting together (orthogonal to each other) as shown in Fig. 6. A plurality of fiber clusters 11 produced as above are "definite fiber aggregates" having a uniform shape and size (for example, the main portion 110 has a rectangular parallelepiped shape). The absorbent member 4 containing the fiber clusters 11 and the water absorbing fibers 12F can be produced, for example, by using a known fiber stacking apparatus with a rotating drum by a common method. Such a fiber stacking apparatus typically includes a rotating drum having, on an outer peripheral face, an accumulating depression and includes a duct having a flow path for conveying raw materials (fiber clusters 11, water absorbing fibers 12F) of an absorbent core 40 to the accumulating depression. While the rotating drum is rotated around a rotating shaft in the drum circumferential direction, the raw materials conveyed on an air flow (vacuum air) generated in the flow path by aspiration from the inside of the rotating drum are fiber stacked on the accumulating depression. The fiber stacking product formed in the accumulating depression through such a fiber stacking process is an absorbent core 40.

The basis weight of the absorbent member 4 can be appropriately adjusted depending on intended use thereof or the like. For example, when an absorbent member 4 is to be used as the absorbent member of an absorbent article such as disposable diapers and sanitary napkins, the basis weight of the absorbent member 4 is preferably 100 $g/m^2$ or more and more preferably 200 $g/m^2$ or more and is preferably 800 $g/m^2$ or less and more preferably 600 $g/m^2$ or less. As described above, the above basis weight of an absorbent member 4 can be applied to an absorbent core 40 as it is.

The present invention has been described on the basis of the embodiments, but the present invention can be appropriately modified without limitation by the embodiments.

For example, in the above embodiments, the absorbent member 4 includes an absorbent core 40 and a core-wrap sheet 41 overlying the absorbent core, but the core-wrap sheet 41 is not necessarily included.

In the absorbent core pertaining to the present invention, all the contained fiber clusters (synthetic fiber aggregates) are not necessarily such definite fiber aggregates as the fiber clusters 11, and in addition the definite fiber aggregates, an extremely small amount of indefinite fiber aggregates may be contained without departing from the scope of the present invention.

The absorbent article of the present invention widely encompasses articles used for absorption of body fluids excreted from a human body (such as urine, loose feces, menstrual blood, and sweat) and encompasses, in addition to the above-described sanitary napkin, sanitary shorts, what are called open type disposable diapers with attachment tapes, pull-on disposable diapers, incontinence pads, and the like. In consideration of the above embodiments of the present invention, the following aspects are further disclosed.

<1> An absorbent member is used to be in direct or indirect contact with the skin, has a skin-facing surface to be placed relatively close to the skin of a user using the absorbent member and a non-skin-facing surface to be placed relatively distant from the skin of the user, includes fiber clusters containing synthetic fibers, and includes water absorbing fibers. In the absorbent member, a plurality of the fiber clusters are interlaced with each other or with the water absorbing fibers, and the content mass ratio of the fiber clusters to the water absorbing fibers is smaller in a side of the skin-facing surface than in a side of the non-skin-facing surface.

<2> The absorbent member as set forth in clause <1>, in which the content mass ratio of the fiber clusters to the water absorbing fibers gradually increases from the side of the skin-facing surface toward the side of the non-skin-facing surface.

<3> An absorbent member is used to be in direct or indirect contact with the skin, has a skin-facing surface to be placed relatively close to the skin of a user using the absorbent member and a non-skin-facing surface to be placed relatively distant from the skin of the user, includes fiber clusters containing synthetic fibers, and includes water absorbing fibers. In the absorbent member, a plurality of the fiber clusters are interlaced with each other or with the water absorbing fibers, and the proportion of the mass of the fiber clusters to the total mass of the fiber clusters and the water absorbing fibers is smaller in a side of the skin-facing surface than in a side of the non-skin-facing surface (i.e. larger in a side of the non-skin-facing surface than in a side of the skin-facing surface).

<4> The absorbent member as set forth in clause <3>, in which the proportion of the mass of the fiber clusters to the total mass of the fiber clusters and the water absorbing fibers gradually increases from the side of the skin-facing surface toward the side of the non-skin-facing surface.

<5> The absorbent member as set forth in any one of clauses <1> to <4>, in which the proportion of the mass of the fiber clusters to the total mass of the fiber clusters and the water absorbing fibers is 0% by mass or more and 60% by mass or less in the skin-facing surface side and is 50% by mass or more and 100% by mass or less in the non-skin-facing surface side, and the difference therebetween is 15% by mass or more.

<6> The absorbent member as set forth in clause <5>, in which the difference is 80% by mass or more.

<7> The absorbent member as set forth in any one of clauses <1> to <6>, in which the fiber cluster includes a main portion formed by two opposite base surfaces and a body surface intersecting the base surfaces.

<8> The absorbent member as set forth in clause <7>, in which the total area of the two base surfaces is larger than the total area of the body surface.

<9> The absorbent member as set forth in clause <7> or <8>, in which the base surfaces have a rectangular shape in a planar view, and the short side of the rectangular shape is equal to or shorter than the thickness of the absorbent member.

<10> The absorbent member as set forth in clause <9>, in which the length of the short side of the base surface is 0.3 mm or more and preferably 0.5 mm or more and is 10 mm or less and preferably 6 mm or less.

<11> The absorbent member as set forth in clause <9> or <10>, in which the length of the long side of the base surface is 0.3 mm or more and preferably 2 mm or more and is 30 mm or less and preferably 15 mm or less.

<12> The absorbent member as set forth in any one of clauses <7> to <11>, in which the number of fiber end portions per unit area in the body surface is greater than the number of fiber end portions per unit area in each of the base surfaces.

<13> The absorbent member as set forth in any one of clauses <7> to <12>, in which the ratio of the number of fiber ends in a unit area on the base surface, $N_1$, to the number of fiber ends in a unit area on the body surface, $N_2$, $N_1/N_2$, is 0 or more and 0.90 or less and preferably 0.05 or more and 0.60 or less.

<14> The absorbent member as set forth in any one of clauses <7> to <13>, in which the number of fiber ends in a unit area on the base surface is 0 or more and 8 or less and preferably 3 or more and 6 or less.

<15> The absorbent member as set forth in any one of clauses <7> to <14>, in which the number of fiber ends in a unit area on the body surface is 5 or more and 50 or less and preferably 8 or more and 40 or less.

<16> The absorbent member as set forth in any one of clauses <7> to <15>, in which the fiber cluster includes an extending fiber portion that contains a fiber extending outward from the main portion and has a lower fiber density than that of the main portion.

<17> The absorbent member as set forth in clause <16>, in which at least one of the extending fiber portions is an extending fiber bundle extending outward from the body surface and containing a plurality of fibers.

<18> The absorbent member as set forth in any one of clauses <1> to <17>, in which the skin-facing surface side contains a water absorbing polymer.

<19> The absorbent member as set forth in any one of clauses <1> to <18>, in which the content mass ratio between the fiber clusters and the water absorbing fibers, the former/the latter, is 20/80 to 80/20.

<20> The absorbent member as set forth in any one of clauses <1> to <19>, in which the synthetic fibers contained in the fiber clusters are non-water absorbing fibers.

<21> The absorbent member as set forth in any one of clauses <1> to <20>, in which the water absorbing fibers are water absorbing cellulose fibers.

<22> The absorbent member as set forth in any one of clauses <1> to <21>, in which the fiber clusters have a water contact angle of less than 90 degrees and preferably 70 degrees or less.

<23> The absorbent member as set forth in clause <22>, in which the fiber clusters have been treated with a hydrophilizing agent.

<24> The absorbent member as set forth in any one of clauses <1> to <23>, in which the absorbent member includes an absorbent core and a core-wrap sheet.

<25> The absorbent member as set forth in any one of clauses <1> to <24>, in which, in the absorbent member, the fiber clusters are bonded to each other or to the water absorbing fibers by interlacement and are capable of being interlaced with each other or with the water absorbing fibers.

<26> The absorbent member as set forth in clause <25>, in which the total number of the fiber clusters bonded by interlacement and the fiber clusters capable of being interlaced is preferably 50% or more, more preferably 70% or more, and even more preferably 80% or more relative to the total number of the fiber clusters in the absorbent member.

<27> The absorbent member as set forth in any one of clauses <1> to <26>, in which preferably 70% or more, more preferably 80% or more of the fiber clusters having a bonded portion to each other or to the water absorbing fibers are bonded by fiber interlacement.

<28> The absorbent member as set forth in any one of clauses <1> to <27>, in which the fiber clusters are derived from a nonwoven fabric.

<29> An absorbent article includes the absorbent member as set forth in any one of clauses <1> to <28>.

Examples

The present invention will next be described more specifically with reference to examples, but the present invention is not limited to the examples.

[Example 1]

A sanitary napkin having substantially the same basic configuration as that of the napkin 1 shown in Fig. 1 was prepared.

As a topsheet, an air-through nonwoven fabric having a basis weight of 30 g/m$^2$ was used, and as the backsheet, a polyethylene resin film having a basis weight of 37 g/m$^2$ (FL-KDJ100nN, manufactured by Daika Kogyo Co., Ltd.) was used. An absorbent member was produced by a common method with a known fiber stacking apparatus by using fiber clusters and water absorbing fibers as the fiber materials of an absorbent core and using a separately prepared core-wrap sheet of tissue paper having a basis weight of 16 g/m$^2$. To produce the fiber clusters, a material fiber sheet was cut into dice in accordance with Fig. 6. The arrangement of fiber materials (fiber clusters, water absorbing fibers) in the absorbent member of Example 1 was, as with the absorbent member 4 shown in Fig. 3, that the fiber cluster occupancy or the fiber cluster ratio gradually increased from the skin-facing surface side of the absorbent member toward the non-skin-facing surface side.

As the material fiber sheet of the fiber clusters, an air-through nonwoven fabric having a basis weight of 21 g/m$^2$ (fiber sheet having fusion bond portions of constituent fibers) and containing, as the constituent fibers, non-water absorbing thermoplastic fibers that had been made from polyethylene and polyethylene terephthalate resins and treated with the composition A described below (having a water contact angle of 68 degrees) was used. As the water absorbing fibers, bleached softwood kraft pulp (NBKP) was used. The fiber clusters (definite synthetic fiber aggregates) used in the absorbent member had a main portion having such a rectangular parallelepiped shape as shown in Fig. 5(a). The short side 111a of the base surface 111 was 0.8 mm, the long side 111b was 3.9 mm, and the thickness T was 0.6 mm. On the base surface 111, the number of fiber ends in a unit area was 3.2 fiber end portions/mm$^2$, and on the body surface 112, the number of fiber ends in a unit area was 19.2 fiber end portions /mm$^2$.

(Formulation of composition A)

- Potassium salt of alkyl phosphate (prepared by neutralization of Gripper 4131 manufactured by Kao Corporation with potassium hydroxide): 25% by mass
- Sodium salt of dialkyl sulfosuccinate (Pelex OT-P manufactured by Kao Corporation): 10% by mass
- Alkyl(stearyl)betaine (Amphitol 86B manufactured by Kao Corporation): 15% by mass
- Polyoxyethylene (addition molar number: 2) stearylamide (Amisol SDE manufactured by Kawaken Fine Chemicals Co., Ltd.): 30% by mass
- Polyoxyethylene-polyoxypropylene modified silicone (X-22-4515 manufactured by Shin-Etsu Chemical Co., Ltd.): 20% by mass

[Example 2]

The same procedure as in Example 1 was performed except that the arrangement of fiber materials (fiber clusters, water absorbing fibers) in the absorbent member was, as with the absorbent member 4A shown in Fig. 4, that two portions different in fiber cluster occupancy or fiber cluster ratio were arranged in the thickness direction, giving a sanitary napkin as Example 2. In other words, the absorbent member in the sanitary napkin of Example 2 included a water absorbing fiber rich portion 12P as the skin-facing surface side and a fiber cluster rich portion 11P as the non-skin-facing surface side. The portions 12P and 11P were bonded to each other on the interface therebetween by interlacement of the constituent fibers, and the interface is located at the center of the absorbent member in the thickness direction.

[Example 3]

The same procedure as in Example 2 was performed except that in the absorbent member, the water absorbing fiber rich portion 12P had a fiber cluster occupancy of 0% by mass, or the skin-facing surface side of the absorbent member had a water absorbing fiber content of 100% by mass, and the fiber cluster rich portion 11P had a fiber cluster content of 100% by mass, or the non-skin-facing surface side of the absorbent member had a water absorbing fiber occupancy of 0% by mass, giving a sanitary napkin of Example 3.

[Example 4]

The fiber cluster in Example 3 was changed to have a base surface 111 having a short side 111a with a length of 0.8 mm and a long side 111b with a length of 5.0 mm and to have a thickness of 0.6 mm. In the changed fiber cluster, the number of fiber ends in a unit area was the same as in Example 2. The same absorbent member as in Example 3 except the above points was used to produce a sanitary napkin as Example 4.

[Comparative Example 1]

The absorbent member of a commercially available sanitary napkin (manufactured by Unicharm Corporation, trade name "Tanom Pew Slim 23 cm") was used as Comparative Example 1. The absorbent member of Comparative Example 1 is a mixture of synthetic fibers and cellulose fibers (water absorbing fibers) and contains no fiber clusters.

[Comparative Example 2]

The same procedure as in Example 1 was performed except that the absorbent member was changed to the following member with reference to Patent Literature 1, giving a sanitary napkin as Comparative Example 2.
The absorbent member used in Comparative Example 2 was the same as the absorbent member used in Example 1 except that indefinite nonwoven fabric pieces were used as the fiber clusters in an absorbent core, and the absorbent core was covered with a core-wrap sheet and then was treated with hot air to fusion bond the nonwoven fabric pieces together contained in the absorbent core. In the hot air process subjected to the absorbent member, a mixed aggregate of nonwoven fabric pieces and pulp fibers (210 mm in length × 66 mm in width) was allowed to stand in an electric dryer (for example, manufactured by Isuzu Seisakusho) at a temperature of 150°C for 600 seconds, and the nonwoven fabric pieces were fusion bonded together. The used indefinite nonwoven fabric pieces were prepared by tearing the same air-through nonwoven fabric as those used in Examples 1 and 2 in any directions, and had a dimension of about 25 mm in a planar view.

[Performance evaluation]

The compression workload in a wet state (w-WC), the twisting rate, and the surface diffusion area of each sanitary napkin of Examples and Comparative Examples were determined by the following methods. For Example 4, the recovery workload in a dry state (d-WC') and the compression distortion rate in a dry state (d-$\Delta T/T_0$) were also determined by the following methods. The results are shown in Table 1.

<Measurement method of compression workload (WC)>

It is commonly known that the compression workload (WC) of a sample can be represented by a value measured by Kawabata Evaluation System (KES) manufactured by Kato Tech Co., Ltd. (reference literature: The Standardization and Analysis of Texture Evaluation (2nd Edition), author: Sueo Kawabata, July 10, 1980). Specifically, Automatic Compression Tester KES-G5 manufactured by Kato Tech Co., Ltd. was used to determine a compression workload (WC) and a compression recovery rate (RC). The measurement procedures are as described below.
An "absorbent article with an absorbent member (sanitary napkin)" as a sample is attached to a test stand of a compression tester. Next, the sample is compressed between steel plates each having a round flat surface with an area of 2 cm². The compression speed is set at 0.02 cm/sec, and the maximum compression load is set at 490 mN/cm². In the recovery process, the measurement was performed at the same speed. The compression workload (WC) is represented by the following equation, and the unit is "mN·cm/cm²". In the equation, $T_m$, $T_o$, and $P_a$ are a thickness at a load of 490 mN/cm², a thickness at a load of 4.9 mN/cm², and a load (mN/cm²) at the time of measurement (compression process), respectively.
The compression workload calculated as above is the compression workload of the sample in a wet state (w-WC). A sample having a larger w-WC value is considered to have higher cushioning properties and is highly evaluated.

[Mathematical Equation 1]

$$WC = \int_{T_0}^{T_m} P_a dT$$

An "absorbent article with an absorbent member in a wet state" as a measurement object of the measurement method is prepared in the following procedure. First, an absorbent article before injection of a defibrinated horse blood is allowed to stand in an environment at a temperature of 23°C and a relative humidity of 50% RH for 24 hours, and an absorbent article in a dry state is prepared. The absorbent article in a dry state is so placed horizontally that the topsheet (skin-facing surface) faces upward, and on the topsheet, an elliptical inlet (a major axis length of 50 mm, a minor axis length of 23 m) is placed. From the inlet, 3.0 g of defibrinated horse blood is injected, then the article is allowed to stand for 1 minute, and subsequently, 3.0 g of defibrinated horse blood is further injected. After the injection, the condition is maintained for 1 minute to give an absorbent article with an absorbent member in a wet state. The defibrinated horse blood injected into the measurement object is the same as that prepared in <Measurement method of surface diffusion area> described later.

<Measurement method of recovery workload (WC')>

The recovery workload (WC') of a sample can be determined by using KES described above. Specifically, Automatic Compression Tester KES-G5 manufactured by Kato Tech Co., Ltd. was used to determine a recovery workload (WC'). The measurement procedure is as described below.

An "absorbent article with an absorbent member (sanitary napkin)" as a sample is attached to a test stand of a compression tester. Next, the sample is compressed between steel plates each having a round flat surface with an area of 2 cm$^2$. The compression speed is set at 0.2 cm/sec, and the maximum compression load is set at 2,450 mN/cm$^2$. In the recovery process, the measurement was performed at the same speed. The recovery workload (WC') is represented by the following equation, and the unit is "mN·cm/cm$^2$". In the equation, $T_m$, $T_0$, and $P_b$ are a thickness at a load of 2,450 mN/cm$^2$, a thickness at a load of 4.9 mN/cm$^2$, and a load (mN/cm$^2$) at the time of measurement (recovery process), respectively.

[Mathematical Equation 2]

$$WC' = - \int_{T_m}^{T_0} P_b dT$$

The recovery workload (WC') is not displayed on a test result screen page of KES-G5, and the test result screen page displays the compression recovery rate (RC) calculated from the compression workload (WC) and the recovery workload (WC'). In such a case, the parameters (WC, RC) displayed by the measurement device are used to calculate the recovery workload (WC') in accordance with the following equation.

[Mathematical Equation 3]

$$WC' = RC \times WC \div 100$$

The "recovery workload in a dry state (d-WC')" is determined by using an "absorbent article in a dry state (sanitary napkin)" as the sample in the <Measurement method of recovery workload (WC')>. The absorbent member included in the absorbent article in a dry state is an unused absorbent member, absorbs no liquid, and is in a dry state. According to the study by the inventors of the present invention, an absorbent member in a dry state having a larger d-WC' value is considered to have higher compression recovery properties and is highly evaluated.

<Measurement method of compression distortion rate ($\Delta T/T_0$)>

The compression distortion rate ($\Delta T/T_0$) of a sample can be determined by using KES described above. Specifically, Automatic Compression Tester KES-G5 manufactured by Kato Tech Co., Ltd. was used to determine a compression distortion rate ($\Delta T/T_0$). The measurement procedure is as described below.

An "absorbent article with an absorbent member (sanitary napkin)" as a sample is attached to a test stand of a compression tester. Next, the sample is compressed between steel plates each having a round flat surface with an area of 2 cm$^2$, and the compression load gradually increases. When the load reaches a predetermined maximum value (maximum load), the thickness (compression thickness) $T_m$ of the measurement object is determined. It should be noted that the measurement object has no wrinkle or folding. Measurement conditions of the compression tester are as described below.

- Compression speed: 0.2 mm/sec
- Maximum load: 2,450 mN/cm$^2$
- SENS: 10
- DEF: 20

The initial thickness ($T_0$) of a measurement object is determined at a load of 103.9 mN/cm$^2$. The compression distortion rate (%) is calculated in accordance with the following equation.

$$\text{Compression distortion rate } (\Delta T/T_0) = \{(T_0 - T_m)/T_0\} \times 100$$

The "compression distortion rate in a dry state (d-$\Delta T/T_0$)" is determined by using an "absorbent article in a dry state (sanitary napkin)" as the sample in the <Measurement method of compression distortion rate ($\Delta T/T_0$)>. The absorbent member included in the absorbent article in a dry state is an unused absorbent member, absorbs no liquid, and is in a dry state. According to the study by the inventors of the present invention, an absorbent member in a dry state having a larger d-$\Delta T/T_0$ value is considered to have higher flexibility and is highly evaluated.

For measurement conditions of the d-WC' and d-$\Delta T/T_0$, the speed was 10 times that in common conditions of KES-G5. According to the study by the inventors of the present invention, such measurement conditions more exactly imitate the movement of a wearer, such as walking and sitting.

<Measurement method of twisting rate>

The twisting rate of a sanitary napkin was evaluated by using a driving human body model of the lower half of a female body. First, the center width (the dimension in the lateral direction at the center in the longitudinal direction of the napkin) (center width before walking) of a napkin as a measurement object was determined, and the napkin was attached to shorts, which were attached to the female body model. Next, the human body model was allowed to walk at a speed of 100 steps/min for 30 minutes. After walking for 3 minutes, 1.5 g of defibrinated horse blood was injected into the attached napkin over 15 seconds. This operation was repeated 6 times during walking of the human body model, and the defibrinated horse blood in a total amount of 9 g was injected into the napkin. The napkin was then detached from the shorts, and the center width (center width after walking) was determined. From the center width before walking and the center width after walking, the twisting rate (%) was calculated in accordance with the following equation. A napkin having a smaller twisting rate value is unlikely to twist and is highly evaluated. The defibrinated horse blood injected into a measurement object is the same as that prepared in <Measurement method of surface diffusion area> described later.

$$\text{Twisting rate} = [\{(\text{center width before walking}) - (\text{center width after walking})\}/(\text{center width before walking})] \times 100$$

<Measurement method of surface diffusion area>

On a slope at an angle of 45° to the horizontal plane, a measurement object (sanitary napkin) is fixed while the skin-facing surface thereof faces the slope. From the skin-facing surface of the measurement object, 1.5 g of defibrinated horse blood is injected over 23 seconds, then the measurement object is allowed to stand for 3 minutes, and the same amount of defibrinated horse blood is injected from the same position over the same time. This defibrinated horse blood injecting and standing operation is repeated 6 times, and the defibrinated horse blood in a total amount of 9 g is injected into the measurement object. After completion of the injecting operation, the diffusion area of the defibrinated horse blood on the skin-facing surface of the measurement object is determined as the surface diffusion area of the measurement object.

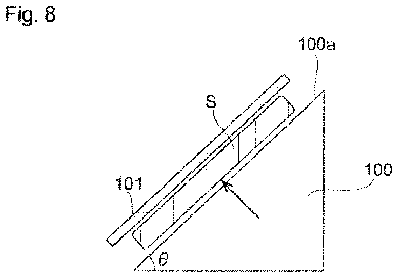

The above <Measurement method of surface diffusion area> will be specifically described with reference to Fig. 8. A measurement object S (sanitary napkin) has a 240 mm $\times$ 75 mm quadrangular shape in a planar view. A measurement table 100 used for the measurement has a slope 100a at an angle $\theta$ of 45° to the horizontal plane. The measurement object S is placed on the slope 100a such that the skin-facing surface thereof faces the slope 100a, and an acrylic plate 101 having a thickness of 3 mm and an area larger than that of the measurement object S is placed on the measurement object S. The defibrinated horse blood injected as a pseudo-blood into the measurement object S has a viscosity of 8 mPa·s determined by using a Brookfield viscometer (manufactured by Toki Sangyo, type TVB-10M, measurement conditions: No. 19 rotor, 30 rpm, 25°C, for 60 seconds). As such a defibrinated horse blood, for example, defibrinated horse blood manufactured by Nippon Bio-test Laboratories Inc. can be used, and the viscosity can be adjusted within the above predetermined range by controlling the blood cell-plasma ratio, as necessary. The injection position of defibrinated horse blood on the measurement object S is the center on the skin-facing surface of the measurement object S (the position indicated by the arrow in Fig. 8), and the injection is performed by using a microtube pump (manufactured by TOKYO RIKAKIKAI Co., Ltd) (not shown). To the pump, a tube (not shown) is connected, and an end of the tube opposite to the end connected to the pump is connected to the measurement object S on the slope 100a. The defibrinated horse blood is injected through the tube into the measurement object S. The diffusion area of defibrinated horse blood on the skin-facing surface of the measurement object S can be determined as follows: a defibrinated horse blood distribution region on the measurement object S is copied on an OHP sheet, and the OHP sheet is processed by a common method with an image analysis software, NexusNewQube (manufactured by Nexus).

Table 1]

| | Example | | | | Comparative Example | |
|---|---|---|---|---|---|---|
| | 1 | 2 | 3 | 4 | 1 | 2 |
| Presence or absence of fiber clusters | Presence | Presence | Presence | Presence | Absence | Presenc e |
| Bonding manner between fiber clusters | Interlaceme nt | Interlaceme nt | Interlaceme nt | Interlaceme nt | - | Fusion bond |
| Outer shape of fiber cluster | Definite shape | Definite shape | Definite shape | Definite shape | | Indefinit e shape |
| Fiber cluster size (mm) (short side x long side of base surface) | 0.8×3.9 | 0.8×3.9 | 0.8×3.9 | 0.8×5 | - | 25*1 |
| Fiber cluster distribution state in absorbent member | Localized in non-skin-fac ing surface side (Fig. 3) | Localized in non-skin-fac ing surface side (Fig. 4) | Localized in non-skin-fac ing surface side (Fig. 4) | Localized in non-skin-fac ing surface side (Fig. 4) | Uniform distribut ed in absorbe nt member | Uniform distribut ed in absorbe nt member |
| Fiber cluster occupancy A in skin-facing surface side of absorbent member (% by mass) | 10 | 10 | 0 | 0 | 0 | 50 |
| Fiber cluster occupancy B in non-skin-facing surface side of absorbent member (% bv mass) | 100 | 90 | 100 | 100 | 0 | 50 |
| Fiber cluster occupancy B-fiber cluster occupancy A (% bv mass) | 90 | 80 | 100 | 100 | 0 | 0 |
| Basis weight of fiber clusters (g/m$^2$) | 175 | 175 | 175 | 175 | 116 | 175 |
| Basis weight of water absorbing fibers (g/m$^2$) | 175 | 175 | 175 | 175 | 116 | 175 |
| Absorbent member thickness (mm) | 5.7 | 5.7 | 5.7 | 5.7 | 2.8 | 5.7 |

(continued)

| | | Example | | | | Comparative Example | |
|---|---|---|---|---|---|---|---|
| | | 1 | 2 | 3 | 4 | 1 | 2 |
| Evaluation | w-WC (mN·cm/cm$^2$) | 90.2 | 85.3 | 90.2 | 90.3 | 22.1 | 64.7 |
| | Twisting rate (%) | 19 | 23 | 19 | 23 | 40 | 25 |
| | Surface diffusion area (cm$^2$) | 34.4 | 34.4 | 34.4 | 30.5 | 34.7 | 62.0 |
| | d-WC' (mN·cm/cm$^2$) | / | / | / | 225.4 | / | / |
| | d-$\Delta T/T_0$ (%) | / | / | / | 64.6 | / | / |
| *1: Average dimension of indefinite fiber clusters | | | | | | | |

As shown in Table 1, each example, in which a plurality of fiber clusters are interlaced with each other or with water absorbing fibers, and the fiber cluster occupancy satisfies the magnitude relation "non-skin-facing surface side > skin-facing surface side", has a larger compression workload w-WC in a wet state than those of Comparative Examples 1 and 2, which do not meet these conditions, and thus has excellent cushioning properties. In addition, each example has a small twisting rate, thus is unlikely to twist, and has excellent liquid drawability due to the above magnitude relation. In particular, comparison of each example with Comparative Example 2 reveals that in order to produce an absorbent member having a large compression workload and excellent cushioning properties even in a wet state, it is effective to use definite fiber clusters and to bond the fiber clusters to each other by interlacement.

Industrial Applicability

The absorbent member of the present invention has high cushioning properties, is unlikely to twist, has excellent liquid drawability, and can improve wearing comfort when included in an absorbent article.
The absorbent article of the present invention includes such a high-quality absorbent member and thus has excellent wearing comfort and excellent leak-proof performance.

**Claims**

1.   An absorbent member used to be in direct or indirect contact with a skin, having a skin-facing surface to be placed relatively close to a skin of a user using the absorbent member and a non-skin-facing surface to be placed relatively distant from a skin of a user, the absorbent member comprising:

    fiber clusters containing synthetic fibers; and
    water absorbing fibers, wherein
    a plurality of the fiber clusters are interlaced with each other or with the water absorbing fibers, and
    a content mass ratio of the fiber clusters to the water absorbing fibers is smaller in a side of the skin-facing surface than in a side of the non-skin-facing surface.

2.   The absorbent member according to claim 1, wherein the content mass ratio of the fiber clusters to the water absorbing fibers gradually increases from the side of the skin-facing surface toward the side of the non-skin-facing surface.

3.   An absorbent member used to be in direct or indirect contact with a skin, having a skin-facing surface to be placed relatively close to a skin of a user using the absorbent member and a non-skin-facing surface to be placed relatively distant from a skin of a user, the absorbent member comprising:

> fiber clusters containing synthetic fibers; and
> water absorbing fibers, wherein
> a plurality of the fiber clusters are interlaced with each other or with the water absorbing fibers, and
> a proportion of a mass of the fiber clusters to a total mass of the fiber clusters and the water absorbing fibers is larger in a side of the non-skin-facing surface than in a side of the skin-facing surface.

4. The absorbent member according to claim 3, wherein the proportion of the mass of the fiber clusters to the total mass of the fiber clusters and the water absorbing fibers gradually increases from the side of the skin-facing surface toward the side of the non-skin-facing surface.

5. The absorbent member according to any one of claims 1 to 4, wherein the proportion of the mass of the fiber clusters to the total mass of the fiber clusters and the water absorbing fibers is 0% by mass or more and 60% by mass or less in the skin-facing surface side and is 50% by mass or more and 100% by mass or less in the non-skin-facing surface side, and a difference therebetween is 15% by mass or more.

6. The absorbent member according to claim 5, wherein the difference is 80% by mass or more.

7. The absorbent member according to any one of claims 1 to 6, wherein the fiber cluster includes a main portion formed by two opposite base surfaces and a body surface intersecting the base surfaces.

8. The absorbent member according to claim 7, wherein a total area of the two base surfaces is larger than a total area of the body surface.

9. The absorbent member according to claim 7 or 8, wherein the base surfaces have a rectangular shape in a planar view, and a short side of the rectangular shape is equal to or shorter than a thickness of the absorbent member.

10. The absorbent member according to any one of claims 7 to 9, wherein the number of fiber end portions per unit area in the body surface is greater than the number of fiber end portions per unit area in each of the base surfaces.

11. The absorbent member according to any one of claims 7 to 10, wherein a ratio of the number of fiber ends in a unit area on the base surface, N1, to the number of fiber ends in a unit area on the body surface, $N_2$, $N_1/N_2$, is 0 or more and 0.90 or less.

12. The absorbent member according to claim 11, wherein the ratio $N_1/N_2$ is 0.05 or more and 0.60 or less.

13. The absorbent member according to any one of claims 7 to 12, wherein the number of fiber ends in a unit area on the base surface is 0 or more and 8 or less.

14. The absorbent member according to any one of claims 7 to 13, wherein the number of fiber ends in a unit area on the body surface is 5 or more and 50 or less.

15. The absorbent member according to any one of claims 7 to 14, wherein the fiber cluster includes an extending fiber portion that contains a fiber extending outward from the main portion and has a lower fiber density than that of the main portion.

16. The absorbent member according to claim 15, wherein at least one of the extending fiber portions is an extending fiber bundle extending outward from the body surface and containing a plurality of fibers.

17. The absorbent member according to any one of claims 1 to 16, wherein the skin-facing surface side contains a water absorbing polymer.

18. The absorbent member according to any one of claims 1 to 17, wherein a content mass ratio between the fiber clusters and the water absorbing fibers, the former/the latter, is 20/80 to 80/20.

19. The absorbent member according to any one of claims 1 to 18, wherein the synthetic fibers contained in the fiber clusters are non-water absorbing fibers.

20. The absorbent member according to any one of claims 1 to 19, wherein the water absorbing fibers are water absorbing cellulose fibers.

21. The absorbent member according to any one of claims 1 to 20, wherein the fiber clusters have a water contact angle of less than 90 degrees.

22. The absorbent member according to claim 21, wherein the fiber clusters have been treated with a hydrophilizing agent.

23. The absorbent member according to any one of claims 1 to 22, wherein the absorbent member includes an absorbent core and a core-wrap sheet.

24. The absorbent member according to any one of claims 1 to 23, wherein, in the absorbent member, the fiber clusters are bonded to each other or to the water absorbing fibers by interlacement and are capable of being interlaced with each other or with the water absorbing fibers.

25. The absorbent member according to claim 24, wherein a total number of fiber clusters bonded by the interlacement and fiber clusters capable of forming the interlacement is 50% or more relative to a total number of the fiber clusters in the absorbent member.

26. The absorbent member according to any one of claims 1 to 25, wherein 70% or more of the fiber clusters having a bonded portion to each other or to the water absorbing fibers are bonded by fiber interlacement.

27. The absorbent member according to any one of claims 1 to 26, wherein the fiber clusters are derived from a nonwoven fabric.

28. An absorbent article comprising the absorbent member according to any one of claims 1 to 27.

Fig. 1

Fig. 2

Fig. 3

4

12F    11(11F)    13

12P

11P

41

11(11F)    12F    40

Y

Fig. 4

4A

13    12F

12P

11P

41

11(11F)    40

Y

Fig. 5(a)    11A(11)

L2

112  111  111b    111a

T

L1

112

111a

111    112  111b

Z

112

Fig. 5(b)

L2

111

11B(11)

T

111    112

Fig. 6

Fig. 7(a)

Fig. 7(b)

Fig. 8

**INTERNATIONAL SEARCH REPORT**

| International application No. |
|---|
| PCT/JP2018/042938 |

A. CLASSIFICATION OF SUBJECT MATTER
Int.Cl. A61F13/534(2006.01)i, D04H1/4382(2012.01)i, D04H1/46(2012.01)i

According to International Patent Classification (IPC) or to both national classification and IPC

B. FIELDS SEARCHED

Minimum documentation searched (classification system followed by classification symbols)
Int.Cl. A61F13/534, D04H1/4382, D04H1/46

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2017/079586 A1 (THE PROCTER & GAMBLE COMPANY) 11 May 2017, page 9, line 23 to page 13, line 5, page 21, line 19 to page 22, line 25, fig. 1-3 & JP 2018-531730 A & US 2017/0119588 A1 & EP 3370784 A1 & CN 108348634 A | 1-28 |
| A | JP 2007-211367 A (KAO CORP.) 23 August 2007, paragraphs [0012], [0028]-[0031], [0035], fig. 2-3 (Family: none) | 1-28 |

☒ Further documents are listed in the continuation of Box C.   ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 14 February 2019 (14.02.2019) | 26 February 2019 (26.02.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2018/042938 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2005-237952 A (KAO CORP.) 08 September 2005, paragraphs [0018], [0023], [0030], fig. 2-3 & US 2008/0287901 A1, paragraphs [0023], [0028], [0035], fig. 2-3 & WO 2005/070363 A1 & EP 1709948 A1 & CN 1913859 A | 1-28 |
| A | JP 2002-301105 A (UNI-CHARM CORP.) 15 October 2002, paragraphs [0025]-[0030], fig. 1, 3 & US 2002/0147434 A1, paragraphs [0030]-[0035], fig. 1, 3 & EP 1247509 A2 & KR 10-2002-0079471 A | 1-28 |
| A | JP 2017-202265 A (UNI-CHARM CORP.) 16 November 2017, paragraphs [0043]-[0044], fig. 4 & WO 2017/195527 A1 | 1-28 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100174259 A
- US 4813948 B
- JP 2003052750 A

**Non-patent literature cited in the description**

- **SUEO KAWABATA.** The Standardization and Analysis of Texture Evaluation. 10 July 1980